# EUROPÄISCHE PATENTSCHRIFT

(11) **EP 3 732 293 B1**
(45) Veröffentlichungstag und Bekanntmachung des Hinweises auf die Patenterteilung: **03.07.2024**
(21) Anmeldenummer: 18857443.8
(22) Anmeldetag: 19.12.2018
(51) Int. Cl.: A61K 39/00, A61P 31/14, A61P 31/16, C12N 15/81, A61K 39/12

(54) **OPTIMIERTES WIRTS-/VEKTORSYSTEM ZUR ERZEUGUNG PROTEKTIVER MONO- UND MULTIVALENTER SUBUNIT-VAKZINE AUF BASIS DER HEFE KLUYVEROMYCES LACTIS**
OPTIMISED HOST-/VECTOR SYSTEM FOR GENERATING PROTECTIVE MONO- AND MULTIVALENT SUBUNIT VACCINES BASED ON THE YEAST KLUYVEROMYCES LACTIS.
SYSTÈME HÔTE / VECTEUR OPTIMISÉ POUR GÉNÉRER DES VACCINS DE PROTECTION SOUS-UNITÉS MONO ET MULTIVALENTS À BASE DE LA LEVURE KLUYVEROMYCES LACTIS.

(30) Priorität: 27.12.2017 DE 102017012109
(43) Veröffentlichungstag der Anmeldung: 04.11.2020
(73) Patentinhaber: VEROVACCiNES GmbH, 06120 Halle/Saale (DE)
(72) Erfinder: HÜHRLIMANN, Hans Caspar, 04177 Leipzig (DE); BEHRENS, Martina, 06120 Halle (Saale) (DE); GEBAUER, Mandy, 19053 Schwerin (DE); BREUNIG, Karin, 14109 Berlin (DE); BEHRENS, Sven-Erik, 06120 Halle (Saale) (DE)
(74) Vertreter: Maikowski & Ninnemann Patentanwälte Partnerschaft mbB
(86) Internationale Anmeldenummer: PCT/DE2018/000379
(87) Internationale Veröffentlichungsnummer: WO 2019/129321

(56) Entgegenhaltungen:
- WO-A1-2013/107436
- WO-A2-2010/054649
- JORRIT-JAN KRIJGER ET AL: "A novel, lactase-based selection and strain improvement strategy for recombinant protein expression in Kluyveromyces lactis, art 112", MICROBIAL CELL FACTORIES,, Bd. 11, Nr. 1, 20. August 2012 (2012-08-20), Seiten 1-12, XP021128994, ISSN: 1475-2859, DOI: 10.1186/1475-2859-11-112
- ARNOLD MARINA ET AL: "Protective Vaccination against Infectious Bursal Disease Virus with Whole Recombinant Kluyveromyces lactis Yeast Expressing the Viral VP2 Subunit", PLOS ONE, PUBLIC LIBRARY OF SCIENCE , Bd. 7, Nr. 9 1. September 2012 (2012-09-01), Seiten e42870.1-e42870.11, XP002695514, ISSN: 1932-6203, DOI: 10.1371/JOURNAL.PONE.0042870 Gefunden im Internet: URL:http://www.plosone.org/article/info%3A doi%2F10.1371%2Fjournal.pone.0042870 [gefunden am 2012-09-14]
- TAKAKO IWATA ET AL: "Efficient secretion of human lysozyme from the yeast, Kluyveromyces lactis", BIOTECHNOLOGY LETTERS, SPRINGER NETHERLANDS, DORDRECHT, Bd. 26, Nr. 23, 1. Dezember 2004 (2004-12-01), Seiten 1803-1808, XP019230808, ISSN: 1573-6776, DOI: 10.1007/S10529-004-4614-9
- MUSTILLI A C ET AL: "Comparison of secretion of a hepatitis C virus glycoprotein in Saccharomyces cerevisiae and Kluyveromyces lactis", RESEARCH IN MICROBIOLOGY, ELSEVIER, AMSTERDAM, NL, Bd. 150, Nr. 3, 1. April 1999 (1999-04-01) , Seiten 179-187, XP027033495, ISSN: 0923-2508 [gefunden am 1999-04-01]
- CONSTANCE MEHLGARTEN ET AL: "Divergent Evolution of the Transcriptional Network Controlled by Snf1-Interacting Protein Sip4 in Budding Yeasts", PLOS ONE, Bd. 10, Nr. 10, 1. Juni 2015 (2015-06-01), Seite e0139464, XP055587956, DOI: 10.1371/journal.pone.0139464
- ALBERT J.J. VAN OOYEN ET AL: "Heterologous protein production in the yeast Kluyveromyces lactis", FEMS YEAST RESEARCH, Bd. 6, Nr. 3, 1. Mai 2006 (2006-05-01), Seiten 381-392, XP055529474, GB, NL ISSN: 1567-1356, DOI: 10.1111/j.1567-1364.2006.00049.x

## Beschreibung

### Gebiet der Erfindung

Die Erfindung betrifft rekombinante *Kluyveromyces lactis* (*K. lactis)* Hefen, die zur hocheffizienten Expression eines oder mehrerer Fremdproteine befähigt sind und zur Verwendung als Vakzin für die Erzeugung einer protektiven Immunantwort gegen Pathogene geeignet sind. Die Erfindung stellt insbesondere *K. lactis* Stämme zur gezielten Klonierung Fremdantigen-codierender Nukleinsäuren in das Hefegenom des *K. lactis* Stammes bereit, die dadurch gekennzeichnet ist, dass der *K*. *lactis* Stamm zusätzlich zum *KlLAC4-Locus* am *KlURA3-20*-Locus (*KLLA0E22771g*) und/oder am *KlMET5-1*-Locus (*KLLA0B03938g*) integrierte Expressionskassetten für Fremdantigene aufweist. Die Erfindung betrifft weiterhin integrative Expressionsvektoren und Verfahren zur Erzeugung der *K*. *lactis* Stämme der Erfindung sowie deren Verwendung als Vakzine.

### Hintergrund der Erfindung

Vakzine werden eingesetzt, um Krankheiten vorzubeugen (präventive Impfstoffe) oder um etablierte Krankheiten zu behandeln (immuntherapeutische Impfstoffe). Präventive Impfprogramme haben in den letzten ca. 100 Jahren wesentlich zur Reduktion von Infektionskrankheiten beigetragen. Immuntherapeutische Impfstoffe werden erst seit etwa 20 Jahren entwickelt und eingesetzt, etwa gegen persistente Infektionen mit Viren, Bakterien oder Parasiten oder gegen kanzerogene Erkrankungen. Ziel der Impfung ist die Induktion einer zellulären (d.h. im Wesentlichen T-Zell und NK-Zellvermittelten) und/oder humoralen (d.h. im Wesentlichen B-Zell/Antikörper-vermittelten) Immunantwort sowie eines immunologischen Gedächtnisses ("*memory*") gegen antigene Komponenten von Pathogenen oder malignen (tumorgenen) Zellen. Klassische Vakzine enthalten den gesamten Krankheitserreger in attenuierter (inaktivierter) oder abgetöteter Form, einschließlich dessen genetischen Materials, d.h. Nukleinsäuren in Form von DNA oder RNA. Diese klassischen Impfstoffe benötigen für die Herstellung meist besondere Sicherheitsvorkehrungen und/oder die Verwendung infizierbarer Organismen und/oder von Zellkulturen; zudem müssen diese Impfstoffe oft aufwendig und unter der Verwendung von Kühlketten gelagert und transportiert werden. Außerdem birgt die Verwendung klassischer Impfstoffe die Gefahr, dass Stoffe aus der Herstellung (z.B. aus dem Versuchstier oder aus der Zellkultur) im geimpften Individuum Nebenwirkungen erzeugen oder dass es zu unerwünschten Reaktivierungen des Erregers kommt. Probleme existieren auch bei der Diagnostik: So können beispielsweise im Fall der Impfung von Nutztieren mit kompletten Erregern geimpfte Tiere nicht von natürlich infizierten Tieren unterschieden werden, so dass Frühwarnsysteme, die auf dem Nachweis von Neuinfektionen basieren, nicht einsetzbar sind. Daher wurden sog. "*subunit*-Vakzine" entwickelt, die nur mit definierten Komponenten des Erregers impfen. Voraussetzung für deren Einsatz ist, dass "Hauptantigene" des jeweiligen Krankheitserregers, bekannt sind. Hauptantigene sind meist Oberflächenbestandteile des Erregers, die vom Immunsystem erkannt werden können, z.B. Proteine einer Virushülle oder eines Viruskapsids. Diese Hauptantigene können auch in Abwesenheit eines kompletten Viruspartikels eine humorale und/oder zelluläre Immunantwort und ein immunologisches Gedächtnis im Wirt gegen das Virus induzieren. Da bei "*subunit-*Vakzinierung" weitere Bestandteile des Erregers fehlen, können durch eine Differenzialdiagnose vakzinierte- von natürlich infizierten Individuen unterschieden werden (*Differentiating Infected from Vaccinated Animals* (DIVA)); entsprechend wird auch von einem "*subunit*-Markervakzin" gesprochen. Nachteile vieler *subunit-Vakzine* sind eine oft aufwändige Herstellung und eine oft unzureichende Immunogenität: Während die Krankheitserreger selbst effizient (mit den oben ausgeführten Einschränkungen) gezüchtet werden können, müssen deren Hauptantigene durch kostenintensive und meist ineffiziente Verfahren gentechnisch hergestellt und aufwändig gereinigt werden. Bei so gewonnenen *subunit*-Vakzinen handelt es sich entsprechend um biologisches Material, das eine geringe Haltbarkeit hat und oft gekühlt gelagert und transportiert werden muss. Aus diesen Gründen beruht ein Großteil der Massenimpfstoffe bei Nutztieren immer noch auf dem klassischen Prinzip, das komplette Krankheitserreger verwendet.

Die weit verbreitete Geflügelkrankheit Infektiöse Bursitis (IBD) wird beispielsweise durch das Virus der Infektiösen Bursitis (IBDV) ausgelöst, ein nicht umhülltes Virus mit einem doppelsträngigen, segmentierten RNA Genom aus der *Birnaviridae* Familie. Die meisten Impfstoffe gegen IBD beruhen auf attenuierten (abgeschwächten) oder inaktivierten Viren. Hier stellt sich jedoch das Problem, dass stark attenuierte nicht inaktivierte "Lebendviren" und auch inaktivierten Viren zwar Schutz gegen durchschnittlich pathogene IBD Viren bieten, dies allerdings nicht der Fall ist bei hoch virulenten (*very virulent*) IBD Virusstämmen (vvIBDV). Bis vor kurzem waren stark virulente, attenuierte Viren (*intermediate hot strains*) gegen vvIBDV protektiv - diese Impfstämme haben aber Nebenwirkungen in der Form, dass es zu einer Immunsuppression durch transiente Schädigungen der B-Zellen in der *Bursa fabricii*, einem lymphatischen Organ (Rautenschlein *et al.* (2005)), kommen kann. Gegen jüngst entdeckte vvIBDV Stämme bieten aber auch diese *intermediate hot* Vakzine keinen vollständigen Schutz (Negash *et al.* (2012); Kasanga *et al.* (2007)). Ein Problem der Impfung mit stark attenuierten Lebendviren ist zudem, dass maternale Antikörper die Virusreplikation und damit die Induktion einer Immunantwort verhindern. Eine wirksame Impfung mit diesen Impfstoffen ist daher erst drei Wochen nach Schlupf möglich (Kumar *et al.* (2000); Rautenschlein *et al.* (2005)).

Influenza-A-Viren gehören beispielsweise zu den weltweit bedeutendsten Viruspathogenen (Short *et al.* (2015); Silva *et al.* (2012)). Influenzaviren gehören zur Familie der *Orthomyxoviridae;* es sind umhüllte Viren mit einzelsträngiger, segmentierter RNA als Genom. Wie die meisten RNA-Viren unterliegen auch Influenzaviren einer hohen Mutationsrate. Insbesondere durch die Reassortierung viraler RNA-Segmente entstehen Virusnachkommen mit neuen genetischen und biologischen Eigenschaften (Short *et al.* (2015)). Aufgrund der rapiden Evolution stellt sich bei Impfungen gegen Influenzaviren insbesondere das Problem, dass existierende Vakzine bei neu entstandenen Virusvarianten nicht "greifen". Entsprechend wird schon seit langer Zeit versucht, Vakzine zu entwickeln, die eine Kreuz- und damit auch Langzeitprotektion gegen verschiedene Influenzavarianten aufweisen (Steel *et al.* (2010); Krammer and Palese (2013); Kirchenbaum and Ross (2014); Berthoud *et al.* (2011)).

Das Virus der bovinen viralen Diarrhoe (BVDV) ist ein weitverbreitetes Pathogen von Paarhufern. Bei BVDV handelt es sich um ein Mitglied des Genus Pestivirus der *Flaviviridae* Familie. Das einzelsträngige RNA-Genom dieser Viren unterliegt ebenfalls einer hohen Mutationsrate. Zudem kann es bei trächtigen Tieren zu einer Infektion des Fetus kommen und aufgrund der Immuntoleranz werden dann persistent infizierte (PI) Tiere geboren. Diese PI Tiere verbreiten das Virus weiter und können bei Mutation des Virus zu 100% an der sog. *mucosal disease* versterben. Auch hier wird schon seit langer Zeit versucht, Vakzine zu entwickeln, die eine Kreuz- und Langzeitprotektion gegen verschiedene BVD Virusvarianten aufweisen (Ridpath (2015)).

Wirksame *subunit-Vakzine* können diese Probleme adressieren bzw. lösen. *Subunits* sind in den meisten Fällen Proteinkomponenten von Krankheitserregern; diese können gentechnisch in verschiedenen Wirtszellen hergestellt werden. Neben dem Darmbakterium *Escherichia coli* sind Säugerzellen oder Insektenzellen, die in Zellkulturen vermehrt werden können, Pflanzenzellen und verschiedene Pilze als Wirtssysteme zur heterologen Proteinexpression etabliert. Mikrobielle Systeme wie Bakterien und Pilze lassen sich besonders kostengünstig im großen Maßstab züchten.

Hefezellen der Hefegattungen *Saccharomyces, Pichia* und *Kluyveromyces* werden bereits seit Dekaden routinemäßig zur Expression von Fremdproteinen eingesetzt. Hefezellen haben gegenüber Bakterien den Vorteil, dass es sich um Eukaryoten handelt, d.h. sie gleichen in vielen Aspekten den tierischen Zellen, und eukaryotische Proteine, d.h. Proteine, die in tierischen Zellen gebildet werden und/oder funktionsfähig sein müssen, können in Hefe in nativer oder nahezu nativer Form kostengünstig hergestellt werden (Bathurst (1994); Gellissen & Hollenberg (1997)). Hefen wurden zunächst nur dazu verwendet, um die Fremdproteine zu produzieren; nach der Expression wurden die Proteine aus den Hefezellen gereinigt und als *subunit-Vakzine* eingesetzt. Erst seit kurzer Zeit wird versucht, Hefen selbst oder Zellfraktionen der Hefen als Vakzine zu verabreichen. "Hefebasierte Vakzine" sind entsprechend Hefepartikel, die immunologisch wirksame Komponenten von Krankheitserregern (Antigene) enthalten und die, nach Applikation (z.B. subkutan, intramuskulär oder oral/mukosal), im Wirtsorganismus eine spezifische Immunantwort gegen diese Antigene und damit auch gegen das Pathogen aus dem diese Antigene stammen, auslösen können. Im gewünschten Fall wird in den vakzinierten Organismen ein immunologisches "*memory*" induziert, das im Falle einer nachfolgenden Infektion ("*challenge*") die Vermehrung und/oder Ausbreitung der entsprechenden Krankheitserreger unterbindet und/oder die pathologischen Auswirkungen der Infektion mildert. Wie oben bereits angesprochen, sind die Antigene meist Strukturproteine des Krankheitserregers, deren codierende Nukleinsäuresequenzen (Antigen-codierende Gene) mit gentechnischen Methoden in Hefezellen eingebracht werden und die Expression eines oder mehrerer solcher Strukturproteine ermöglichen. Die so erzeugten rekombinanten Hefen in lebender Form (Hefezellen), nach Abtötung und Trocknung in Pulverform (Hefepartikel) oder nach Zellaufschluss und Homogenisierung (Hefelysat) stellen hefebasierte Vakzine dar. Nach Applikation der Vakzine werden die Antigene vom Immunsystem erkannt und rufen eine humorale und/oder zelluläre Immunabwehr hervor.

Die hefebasierte Vakzinierung ist dem Fachmann aus dem Stand der Technik bekannt. Eine Reihe von US-Patentanmeldungen und -patenten, so z.B. US 20090304741 A1, US 5830463 A, US 7465454 B2 und US 20070166323 A1 beschreiben die Verwendung von *Saccharomyces cerevisiae* (*S. cerevisiae*) Stämmen, die mindestens ein rekombinantes Antigen enthalten, in der Immuntherapie. Es wurde gezeigt, dass diese Hefen wirksam sind, um eine Immunreaktion, insbesondere eine Zell-vermittelte Immunreaktion, zu stimulieren.

WO 2006044923 offenbart Hefe (*S*. *cerevisiae)*, die verschiedene Proteine des Hepatitis C Virus (HCV) rekombinant exprimiert und die eine Immunreaktion, vor allem eine T-Zell-Antwort, gegen diese HCV-Proteine auslösen kann und als Vakzin gegen chronische Hepatitis C eingesetzt werden soll.

WO 2007092792 beschreibt den möglichen Einsatz rekombinanter *S*. *cerevisiae* Hefen gegen Influenzavirus-Infektionen, wobei eine Kombinationen verschiedener Hefestämme benutzt wird, deren Applikation zu einer T-Zell-Induktion, also zu einer zellulären Immunantwort führt.

WO 20101054649 und WO 2013107436 beschreiben den Einsatz von Stämmen der Spezies *Kluyveromyces lactis,* die definierte Antigene enthalten, zur Erzeugung einer protektiven humoralen Immunantwort nach oraler/mukosaler oder subkutaner Applikation ganzer abgetöteter Hefezellen. Die letztgenannten Patente enthalten Anwendungsbeispiele, in denen rekombinante *K. lactis* Stämme, die vom Ausgangsstamm VAK367-D4 abgeleitet wurden, erfolgreich zur Vakzinierung eingesetzt wurden.

Die Möglichkeit der Anwendung rekombinanter *Kluyveromyces lactis* Hefen zur Vakzinierung ist dem Fachmann aus dem Stand der Technik bekannt: (Arnold *et al.* (2012)); WO 20101054649 und WO 2013107436). In Anwendungsbeispielen konnte gezeigt werden, dass durch die subkutane Verabreichung der Hefe *K. lactis,* die das VP2-Kapsidprotein des Virus der Infektiösen Bursitis (IBDV) intrazellulär über eine durch den *LAC4*-Promoter gesteuerte Expressionskassette exprimiert, eine humorale Immunantwort ausgelöst wird, die einen wirksamen Schutz gegen Virusinfektion verleiht. Dies konnte für ein IBD Virus mittlerer Pathogenität gezeigt werden, bis dato jedoch nicht gegen *very virulent* IBDV (vvIBDV). Die früheren Daten zeigten, dass die Wirksamkeit eines Hefevakzins durch Steigerung der intrazellulären Konzentration des viralen Antigens erhöht werden kann (Arnold *et al.* (2012)). Eine technische Variante, um eine Steigerung der Antigenkonzentration zu erreichen, besteht darin, eine zusätzliche Kopie des Transkriptionsaktivatorgens *KlGAL4-1* (alias *LAC9-1*) mittels Integration des pLl-1-Plasmides (Krijger *et al.* (2012) und WO 2013107436) in den IBDV-VP2-exprimierenden Stamm einzubringen (hinterlegte Stämme DSM 25406 und DSM 25407). Die Generierung solcher *K*. *lactis* Vakzinstämme basierte also bislang auf zwei genetischen Eingriffen: Erstens auf der Integration des Antigen-codierenden Fremdgens, zweitens auf der Integration des *KlGAL4-1* Gens. In der bisher praktizierten Form führte letzteres jedoch regelmäßig auch zur Integration von Tandem-Wiederholungen des Plasmids, was nicht nur cytotoxische Effekte aufgrund der starken Überexpression des Aktivators zur Folge hat (Breunig 1989), sondern auch zu unterschiedlichen Kopienzahlen für die *KlGAL4-1-* und *ScURA3*-Gene in auf diese Weise generierten Vakzinstämmen führt.

Die in den oben genannten Anwendungsbeispielen (Arnold *et al.* (2012); WO 20101054649 und WO 2013107436) beschriebene Strategie, die Expression des Fremdgens über einen unmodifizierten *LAC4*-Promoter vorzunehmen, hat den Nebeneffekt, dass auch unter nicht-induzierenden Bedingungen eine minimale Expression des Fremdgens erfolgt, d.h. der Promoter zu einem gewissen Anteil durchlässig ist. Bei Erhöhung der *KlGAL4*-1-Gendosis ist dieser Effekt nochmals deutlich ausgeprägter. Entsprechend kann bei Proteinen, die bei heterologer Expression einen zellschädigenden Effekt (cytopathischen Effekt, CPE) auf die Hefezelle haben, die Biomasse-Bildung bei der Anzucht, z.B. während eines Fed-Batch-Fermentationsprozesses, massiv einschränkt sein. Speziell für diese Fälle müssen alternative Wege gefunden werden, die Genexpression unter nicht-induzierenden Bedingungen möglichst niedrig zu halten.

Verschiedene *subunit-Vakzine* sind erst dann effektiv wirksam, wenn nicht nur eine, sondern mehrere *subunits* eines Pathogens zur Impfung eingesetzt werden. Durch den Einsatz mehrerer Antigen-subunits bei der Impfung kann zudem die Kreuzprotektivität gegen verschiedene Varianten eines Pathogens massiv erhöht werden. Die Ko-Expression gleicher oder verschiedener Antigene kann auch dazu genutzt werden, die Antigenkonzentration in der Hefezelle nochmals zu steigern bzw. ein Vakzin zu erzeugen, das gegen verschiedene Pathogene schützt.

Die zuvor diskutierten Stämme sind in der Regel auxotrophe Stämme, die in Vollmedium häufig schlechter wachsen als prototrophe Stämme. Entsprechend kann eine schnell durchführbare Umwandlung auxotropher Hefestämme in eine prototrophe Form zu verbesserten Wachstumseigenschaften führen.

### Beschreibung der Erfindung:

Die Aufgabe der Erfindung bestand nunmehr darin, neue *K. lactis* Vakzinstämme zur Verfügung zu stellen, mit denen die Nachteile des Standes der Technik überwunden werden können. Insbesondere sollten rekombinante *K. lactis* Stämme bereitgestellt werden, die eine limitierte Kopienzahl des *KlGAL4-1*-Gens, integriert an einer definierten Stelle im Genom, enthalten. Zudem sollten Stämme bereitgestellt werden, die unter nicht-induzierten Bedingungen keine oder eine nur geringe Fremdproteinexpression zulassen, die Expression mehrerer Kopien eines Antigens bzw. die Expression mehrerer Antigene in einer Hefezelle zulassen, die für die Anzucht besser geeignet sind und in effektiver Weise zur protektiven Vakzinierung gegen Pathogene einsetzbar sind. Dabei sollten heterologe Gene, die immunmodulatorisch wirksame Proteine (Antigene) codieren, an definierten Stellen des *K. lactis* Genoms integriert werden. Bei der Selektion der gesuchten Klone mit Fremdgenintegration sollten keine Resistenzgene als Selektionsmarker zum Einsatz kommen. Zudem sollten prototrophe Stämme über ein möglichst einfaches Verfahren aus auxotrophen Stämmen generiert werden. Dies sollte auch die vereinfachte Fermentation der erzeugten Hefevakzinstämme in nicht supplementiertem, synthetischem Medium ermöglichen.

Diese Aufgaben wurden gelöst durch die Bereitstellung eines modularen Systems, das neue Vektoren und neue, gentechnisch veränderte Varianten der Hefe *K. lactis* enthält und das die Generierung von Impfstämmen erlaubt, die auf die spezifischen Eigenschaften der Proteinantigene optimiert sind. Über einen bausteinartigen Austausch von DNA-Elementen zwischen den Vektoren wurde dabei eine effiziente, routinemäßige Klonierung von Fremdantigen-codierenden Bereichen in das Hefegenom erreicht, unabhängig von dem zu exprimierenden Fremdgen. Durch die gezielte genomische Integration der relevanten Fremdgene sind die Hefestämme über sehr viele Generationen stabil und genetisch exakt definiert. Aufgrund dieser Eigenschaften verlaufen unter nicht selektiven Bedingungen Fermentationsprozesse reproduzierbar und können standardisiert werden. Die Optimierung der erfindungsgemäßen *K*. *lactis* Hefen bestand dabei darin, die Proteinproduktionsrate so zu steuern, dass diese möglichst hoch ist, jedoch derart, dass sie unterhalb einer Schwelle liegt, bei der cytopathische Effekte der Antigene den effizienten Fermentationsprozess massiv stören. Dies wurde erreicht durch einen genetischen Eingriff oder durch eine Kombination von mehreren genetischen Eingriffen:
i. die Erhöhung der Konzentration des Lactose-induzierbaren Transkriptionsaktivators,
ii. die gezielte Veränderung des *LAC4*-Promotors, und/oder
iii. die schrittweise Erhöhung der Gendosis für das antigen-codierende Fremdgen.

Die Optimierung der erfindungsgemäßen *K. lactis* Hefen bestand darüber hinaus darin:
iv. mehrere, neue Integrationsstellen für Fremdgen-codierende Kassetten im Hefegenom zu etablieren, um gleichzeitig mehrere Antigene exprimieren zu können.

In einer bevorzugten Ausführungsform wird die Aufgabe der Erfindung gelöst durch Bereitstellung eines *K*. *lactis* Stamms zur gezielten Klonierung Fremdantigen-codierender Nukleinsäuren in das Hefegenom des *K*. *lactis* Stammes, gekennzeichnet dadurch, dass der *K*. *lactis* Stamm zusätzlich zum *KlLAC4-Locus* am *KlURA3-20*-Locus (*KLLA0E22771g*) und/oder am *KlMETS-1-*Locus (*KLLA0B03938g*) integrierte Expressionskassetten für Fremdantigene aufweist. Besonders bevorzugt ist es, wenn der *K*. *lactis* Stamm zusätzlich zum *KlLAC4-Locus* am *KlURA3-20*-Locus (*KLLA0E22771g*) und am *KlMETS-1*-Locus (*KLLA0B03938g*) integrierte Expressionskassetten für Fremdantigene aufweist. Derart veränderte *K. lactis* Stämme haben den Vorteil, dass Gene für die Expression von Fremdgenen an festgelegten, definierten Loci im *K. lactis* Genom integriert werden und die Kopienzahl der Fremdgene steuerbar ist. Darüber hinaus ermöglichen diese *K. lactis* Stämme die Integration verschiedener Gene für die Expression verschiedener Fremdantigene an definierten Loci im *K. lactis* Genom.

Mit "Fremdantigene" bzw. "Fremdproteine" im Sinne dieser Erfindung sind alle Peptide, Polypeptide und Proteine gemeint, die geeignet sind, eine Immunantwort, bevorzugt eine protektive Immunantwort, im Menschen oder in einem Tier gegen ein Pathogen oder kanzerogen entartete Zellen zu erzeugen. Fremdproteine können von Krankheitserregern oder Tumoren jeglicher Art stammen, für die Antigene charakterisiert wurden, die allein in der Lage sind, eine protektive Immunantwort, vorzugsweise eine protektive Immunantwort, zu induzieren.

In einer bevorzugten Ausführungsform stammen die Fremdproteine von Krankheitserregern (Viren, Bakterien, Parasiten), für die Antigene charakterisiert wurden, die allein in der Lage sind, eine protektive Immunantwort, vorzugsweise eine protektive humorale Immunantwort zu induzieren.

Dies sind zum Beispiel:

### Fremdproteine, die von Parasiten stammen

*Necator americanus; Ancylostoma duodenale*: ASP-Protein, *Haemoglobin-abbauende Proteasen*
*Leishmania*: gp63, 46 kD *Promastigot*-*Antigen*, LACK
*Plasmodium*: CSP-Protein, CSA-1, CSA-3, EXP1, SSP2, STARP, SALSA, MSP1, MSP2, MSP3, AMA-1, GLURP, Pfs25, Pfs 28, Pvs25, Pvs 28, Pfs 48/45, Pfs 230
*Schistosoma*: TP1, Sm23, ShGSTs 26 und 28, Paramyosin, *Parasiten-Myosin*, Sm14 Fremdproteine, die von Bakterien stammen
*Mycobakterium tuberculosis*: Ag85A, Hsp65, R8307, 19 kD, 45 kD, 10.4
*Heliobacter pylori*: VacA, LagA, NAP, hsp, Urease, Katalase
Group A *Strepptococcus*: M, SCPA Peptidase, Exotoxine SPEA und SPEC, *Fibronectin binding protein*
*Strepptococcus pneumonia*: PspA, PsaA, BHV 3, BHV 4
*Salmonella typhimurium*: Vi-Antigen
*Shigella*: LPS
*Vibrio cholera*: CTB
*Escherichia coli* ETEC: LT, LT-ST, CTB
*Yersinia pestis*: F1, V

### Fremdproteine, die von Tumorzellen/Tumoren stammen (Tumor-assoziierte Antigene, TAA)

CEA
5T4
MUC1
MART1
HER-2

### Insbesondere bevorzugt sind Fremdproteine, die von Viren stammen.

*Caliciviridae* (Norwalk, HEV): NV 60 kD, HEV ORF2
*Reoviridae* (Rota): VP7, VP4
*Retroviridae* (HIV): Gag, Pol, Nef, Env, gp160, gp120, gp140, gp41
*Flaviviridae* (genus Flavivirus: WNV, Dengue, YF, TBE, JEV): preM-Env, NS3, NS4, NS5
*Flaviviridae* (genus Pestivirus BVDV, CSFV, BDV. Genus Hepacivirus HCV): E1, E2, E^{RNS} (Pesti), C, NS3, NS4, NS5
*Hepadnaviridae* (HBV): HBS Antigen
*Paramyxoviridae* (*Paramyxovirinae*:PIV-1, PIV-2, Mumps,Sendai, PIV-2, PIV-4, Morbilli ): M, HN, N, F
*Paramyxoviridae* (*Pneumovirinae*: RSV): F, G, SH, M
*Rhabdoviridae* (Rabies): G
*Herpesviridae* (EBV, HSV2): gp350/220 (EBV), gB2, gD2 (HSV)
*Coronaviridae* (SARS): CoV, N, M, S
*Orthomyxoviridae* (Influenza A, B): HA, NA, M1, M2, NP
*Papillomaviridae*: L2, E6, E7

In einer weiteren Ausführungsform der Erfindung sind die veränderten *K. lactis* Stämme dadurch gekennzeichnet, dass die Expressionskassetten den *K. lactis LAC4-*12-Promotor (P*LAC4-12*) bzw. Varianten dieses Promotors, den ORF des zu exprimierenden Antigens und den *Ag*TEF1-Terminator enthalten. Diese Ausführungsform hat den Vorteil, dass die Expression von Fremdgenen unter Kontrolle des P*LAC4-12*-Promotors nach Integration am *LAC4-* und/oder *KlURA3-* und/oder *KlMET5*-Locus etwa gleich stark durch Lactose induziert werden.

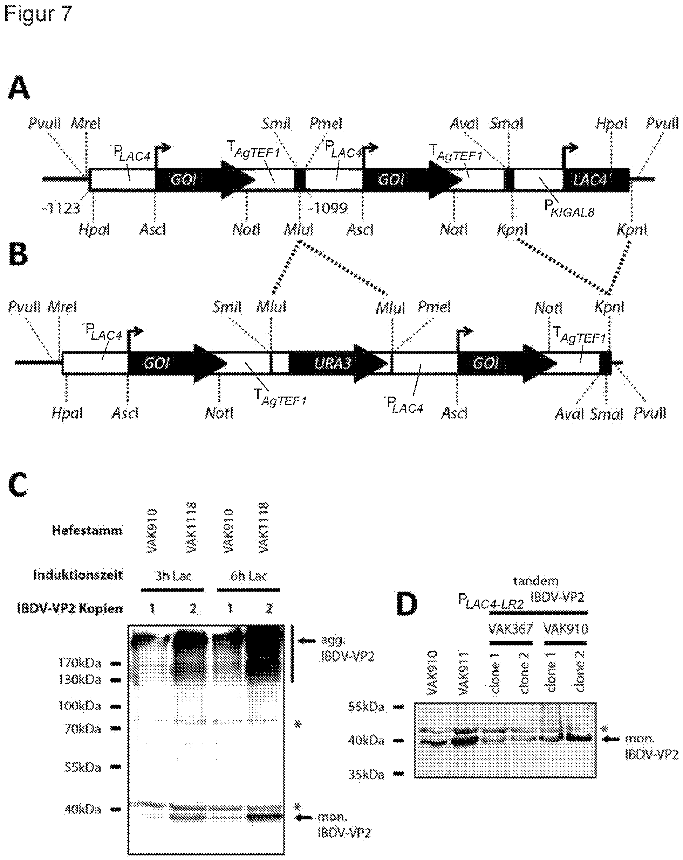

Wie oben beschrieben, besteht eine positive Korrelation zwischen der Antigenkonzentration in Vakzinstämmen und der immunogenen Wirkung des Hefevakzins im Zielorganismus. Um einen CPE bei zu starker Überexpression, beispielsweise durch Integration eines zusätzlichen *KlGAL4-Gens* zu verhindern, kann alternativ das zuvor beschriebene Vektorsystem modifiziert werden, um schnell und effizient mehrere Genkopien hintereinander zu schalten und diese Expressionskassette in einem Schritt an einen der drei Genloci einzubringen (siehe Beispiel 5 und Figur 7A).

In einer vorteilhaften Weiterentwicklung der Erfindung enthalten deshalb die veränderten *K. lactis* Stämme zusätzlich zum *KILAC4*-Locus am *KlURA3-20*-Locus und/ oder am *KlMET5-1-Locus* mehrere Kopien einer Fremdantigen-codierenden Nukleinsäuresequenz, die über Tandem- oder Mehrfach- Expressionskassetten inseriert sind. Diese Expressionskassetten umfassen mehrfache Kopien der jeweils durch den *LAC4*-12-Promotor (P*LAC4-12*) bzw. Varianten dieses Promotors und den *AgTEF1*-Terminator flankierten Antigen-codierenden Regionen (Gene). Durch so vorgenommene Vervielfachung der Genkopien des Antigens kann dessen Expression über einen der jeweiligen Genloci signifikant gesteigert werden.

In einer bevorzugten Ausführungsform die nicht Teil der Erfindung ist, liegt am Locus *KiLAC4* des *K*.

*lactis* Stammes das Gen des Fremdantigens IBDV-VP2 in Form einer Tandem-Expressionskassette vor. Dieser *K*. *lactis* Stamm hat gegenüber Stämmen mit Einfachkopie des Gens, das für das Fremdantigen IBDV-VP2 codiert, den Vorteil, dass das Fremdantigen IBDV-VP2 mit erhöhter Quantität exprimiert wird. Besonders bevorzugt gemäß dieser Ausführungsform der Erfindung ist der Stamm VAK1118 (DSM 32701), der das Gen des Fremdantigens IBDV-VP2 in Form einer Tandem-Expressionskassette am Locus *KiLAC4* aufweist.

Es ist weiterhin bevorzugt, wenn am *KlLAC4-Locus* und/oder am *KlURA3-20*-Locus und/oder am *KlMET5-1*-Locus der erfindungsgemäßen *K. lactis* Stämme eine oder mehrere Kopien verschiedener Fremdantigen-codierender Nukleinsäuren über Einfach-, Tandem- oder Mehrfach- Expressionskassetten inseriert sind. Dadurch können einerseits verschiedene Fremdantigene und andererseits diese verschiedenen Fremdantigene in unterschiedlichen Konzentrationen in der Hefezelle exprimiert werden. Besonders bevorzugt gemäß dieser Ausführungsform ist ein *K. lactis* Stamm, in dem an den *KlLAC4-* und *KlURA3-20-*Loci des *K. lactis* Stammes die codierenden Nukleinsäuresequenzen der Fremdantigene Influenza A HA (A/Puerto Rico/8/1934(H1 N1)) und Influenza A M1 (A/Puerto Rico/8/1934(H1 N1)) inseriert sind und exprimiert werden. Insbesondere bevorzugt gemäß dieser Ausführungsform der Erfindung ist der Stamm VAK1283 (DSM 32697), in dem an den *KlLAC4-* und *KlURA3-20-* Loci des *K*. *lactis* Stammes die codierenden Nukleinsäuresequenzen der Fremdantigene Influenza A HA (A/Puerto Rico/8/1934(H1 N1)) und Influenza A M1 (A/Puerto Rico/8/1934(H1 N1)) inseriert sind.

Wie erwähnt, ist bekannt, dass die Erhöhung der *KlGAL4*-Gendosis zur Steigerung der Antigenproduktion führen kann (Krijger *et al.* 2012 und WO 2013107436). Die Nachteile, dies über die Integration des KlGAL4-exprimierenden pLl-1-Plasmids in einem Zwei-Schritt-Prozess zu erreichen, sind oben aufgeführt. Diese Nachteile wurden erfindungsgemäß dadurch überwunden, dass ein stabiler Ausgangsstamm für die Integration von Fremdgenen bereitgestellt wird, der eine zweite Kopie des *KlGAL4-*Gens enthält. Damit wird sichergestellt, dass alle abgeleiteten Stämme den gleichen genetischen Hintergrund besitzen und exakt eine zusätzliche *KlGAL4*-Genkopie in diesen Stämmen vorliegt. Dies verringert die Cytotoxizität, die bei Expression von multiplen Kopien beobachtet wurde und reduziert die Schritte bei der Vakzinstamm-Herstellung auf lediglich einen Schritt. Außerdem wird die genetische Stabilität erhöht, da die reversible Integration/Exzision des Plasmids entfällt. Die Herstellung eins solchen Stammes kann beispielsweise wie in Beispiel 1 beschrieben erfolgen.

In einer weiteren vorteilhaften Ausführungsform der Erfindung wird somit ein *K. lactis* Stamm bereitgestellt, der zusätzlich zum genomischen *KlGAL4*-Gen noch eine zweite ektopische Kopie des *KlGAL4-Gens* enthält. In diesem Stamm kann die Expression des KlGAL4-Transkriptionsaktivators maximal auf das Doppelte erhöht und die Expression der in den *KlLAC4-Locus* und/oder den *KlURA3-20-* und/oder den *KlMET5-1*-Locus inserierten Fremdgenen über den *LAC4*-12-Promotor bzw. über unten beschriebene Varianten dieses Promotors definiert gesteigert werden. In der herkömmlichen Praxis wurden Plasmide, die KIGAL4 codieren, transient und in vielfacher, unkontrollierter Kopienzahl in die Zelle eingebracht. Dadurch wurde das Fremdantigen oftmals in so hoher Konzentration exprimiert, dass dies zu cytotoxischen Effekten führte. Cytotoxische Effekte können bei den *K. lactis* Stämmen dieser Ausführungsform der Erfindung mit hoher Effektivität herabgesetzt bzw. vermieden werden. Weitere Genloci, die für den gleichen Zweck (Inserierung einer *LAC4-*gesteuerten Expressionskassette) in Zukunft erschlossen werden, können so ebenfalls angesteuert werden. Es hat sich als vorteilhaft erwiesen, wenn die ektopische Kopie des *KlGAL4-Gens,* die durch den *KlGAL4*-Promotor und *KlGAL4-*Terminator flankiert wird, in dem *K. lactis* Stamm an dem Genort *KLLA0E13795g* (*Klavt3::KIGAL4-1,* SEQ ID Nr.: 1) integriert ist. Insbesondere bevorzugt, aber nicht Teil der Erfindung, gemäß dieser Ausführungsform der Erfindung ist der Stamm VAK1111 (DSM 32696), der diese Eigenschaften aufweist.

In einer weiteren bevorzugten Ausführungsform, die nicht Teil der Erfindung ist, wird ein *K. lactis* Stamm bereitgestellt, in dem am Locus *KlLAC4* die codierende Nukleinsäuresequenz des Fremdantigens IBDV-VP2 vorliegt. Insbesondere bevorzugt gemäß dieser Ausführungsform der Erfindung ist der Stamm VAK1171 (DSM 32699). Dieser Stamm enthält zusätzlich eine zweite, ektopische Kopie des *KlGAL4-Gens,* an dem ebenfalls die codierende Nukleinsäuresequenz des Fremdantigens IBDV-VP2 vorliegt. Dieser Stamm zeigt eine gegenüber Stämmen ohne zusätzliche ektopische Kopie des *KlGAL4-*Gens erhöhte Expression des Fremdantigens IBDV-VP2.

Die heterologe Proteinproduktion in Mikroorganismen ist dann problematisch, wenn dies zu einem cytopathischen Effekt (CPE) führt. Die Erfindung stellt daher einen Weg bereit, die Antigenproduktionsphase von der Biomasse-Akkumulationsphase zu entkoppeln. Durch den induzierbaren *LAC4*-Promotor ist dies z.B. durch einen *Fed-*Batch-Fermentationsprozess partiell möglich, wird aber erschwert, da der Promotor P*_{LAC4-12}* unter nicht-induzierenden Bedingungen nicht vollständig stillgelegt (d.h. zu einem gewissen Maß durchlässig) ist. Bei Antigenen mit sehr starkem CPE tritt dadurch eine Reduktion der Wachstumsrate und eine Induktion der zellulären Stressantwort auf, mit nachteiligen Effekten für die Antigenproduktion. Durch die Verdoppelung der *KIGAL4*-Gendosis und/oder die Erhöhung der Anzahl Antigencodierender Sequenzen (s.u.) wird dieses Problem verschärft.

Eine vorteilhafte Weiterentwicklung der *K*. *lactis* Stämme gemäß der Erfindung besteht daher darin, dass die *K. lactis* Stämme eine veränderte Promotorstruktur des *LAC4-12*-Promotors aufweisen, die unter nicht-induzierenden Bedingungen keine oder eine nur geringe Fremdproteinexpression zulässt. Die veränderte Struktur des *LAC4-12-*Promotors ist insbesondere dadurch gekennzeichnet, dass die *basal control region* (BCR) des Promotors P*LAC4-12* zwischen den Positionen-1065 und -1540 (LR2-Deletion; P*LAC4-12-LR2*; SEQ ID Nr.: 2) deletiert ist (siehe hierzu auch Beispiel 2). Wie bereits oben beschrieben, weist diese Ausführungsform der Erfindung den Vorteil gegenüber der herkömmlichen Praxis auf, dass cytotoxische Effekte, die herkömmlich durch zu starke Expression der Fremdgene verursacht werden konnten, mit hoher Effektivität herabgesetzt bzw. vermieden werden. Bevorzugt gemäß dieser Ausführungsform sind *K. lactis* Stämme, in denen am Locus *KlLAC4,* die codierende Nukleinsäuresequenz des Fremdantigens Influenza A HA (A/Puerto Rico/8/1934(H1 N1)) vorliegt. Insbesondere bevorzugt gemäß dieser Ausführungsform, die nicht Teil der Erfindung ist, ist der Stamm VAK1243 (DSM 32702). Dieser Stamm enthält eine LR2-Deletion im LAC4-12-Promotor.

Der *K*. *lactis* Stamm kann auch eine veränderte Struktur des LAC4-12-Promotors aufweisen, die eine Modulation der Fremdproteinexpression ermöglicht, wobei die Anzahl der Bindungsstellen für den Aktivator KIGal4 des Promotors ("*upstream activating sequences"* 1, 2 und 4, 5) variiert und entweder 1, 2, 3 oder 4 KIGal4-Bindungsstellen vorhanden sind. Auf diese Weise können verschiedene Fremdproteine in unterschiedlicher Konzentration (*quality by design*) in einer Hefezelle exprimiert werden. Die verkürzten Promotorvarianten sind unter anderem wichtig für die Modularität des Systems, um beispielsweise Proteine im selben Stamm in optimalen stöchiometrischen Verhältnissen, beispielsweise für die Bildung von hoch immunogenen *virus-like particles* (VLPs), zu exprimieren. Bevorzugt gemäß dieser Ausführungsform der Erfindung ist es, wenn am Locus *KILAC4* des *K*. *lactis* Stammes die codierende Nukleinsäuresequenz des Fremdantigens IBDV-VP2 inseriert ist. Insbesondere bevorzugt gemäß dieser Ausführungsform, die nicht Teil der Erfindung ist, ist der Stamm VAK1131 (DSM 32700). Dieser Stamm enthält eine LR2-Deletion und eine Deletion der *upstream activating sequences* 4 und 5 im LAC4-12-Promotor.

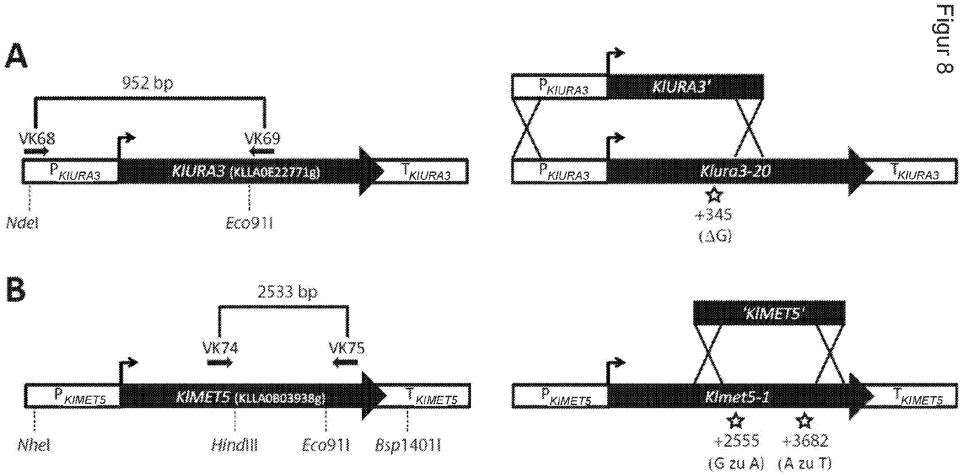

Ein Teil der Aufgabe der Erfindung bestand darin, *K. lactis* Stämme bereitzustellen, die für die Anzucht besser geeignet sind. Dieses Problem wird dadurch gelöst, dass in den erfindungsgemäßen *K. lactis* Stämmen die Genfunktion der Allele *Kllac4, Klura3-20* und *Klmet5-1* wiederhergestellt ist. Die daraus resultierenden *K. lactis* Stämme sind prototroph (Beispiel 6, Fig. 8). Die Fermentation der Vakzinstämme wird so vereinfacht, die Etablierung der Produktionsprozesse erleichtert und kosteneffizienter gemacht. Bevorzugt gemäß dieser Ausführungsform der Erfindung sind *K. lactis* Stämme, in denen an den Loci *KlLAC4, KlURA3-20* und *KlMet5-1* des *K*. *lactis* Stammes in die die codierende Nukleinsäuresequenzen der Fremdantigene BVDV E2 *ectodomain* (Typ 1, CP7), BVDV E2 *ectodomain* (Typ 2, New York 93) und BVDV Npro-NS3 (Typ1, CP7) inseriert sind. Insbesondere bevorzugt gemäß dieser Ausführungsform der Erfindung ist der Stamm VAK1400 (DSM 32698). Dieser Stamm ist prototroph.

In einer besonders bevorzugten Ausführungsform betrifft die Erfindung einen *K. lactis* Stamm, der ausgewählt ist aus den Stämmen

| | |
|---|---|
| VAK1283 | DSM 32697; |
| VAK1395 | DSM 32706; |
| VAK1400 | DSM 32698 |

Diese Stämme wurden am 24.11.2017 bzw. 01.12.2017 (DSM 32705, DSM 32706) bei der Deutsche Sammlung von Mikroorganismen und Zellkulturen GmbH, DSMZ, Inhoffenstrasse 7B, 38124 Braunschweig, Deutschland, entsprechend dem Budapester Vertrag unter den oben angegebenen Nummern hinterlegt

In einem weiteren Aspekt stellt die Erfindung integrative Expressionsvektoren bereit, mit deren Hilfe die *K. lactis* Stämme der Erfindung herstellbar sind.

In einer bevorzugten Ausführungsform, die nicht Teil der Erfindung ist, werden die integrativen Expressionsvektoren *KI*pURA3 (SEQ ID Nr.: 3) und *KI*pMET5 (SEQ ID Nr.: 4) bereitgestellt.

Diese Vektoren enthalten den *LAC4*-12-Promotor (P*LAC4-12*) oder Varianten dieses Promotors (wie für die *K*. *lactis* Stämme oben beschrieben) inklusive dem ORF des zu exprimierenden Antigens, darüber hinaus die *AgTEF1*-Terminatorsequenz sowie *targeting sequences,* die eine gezielte Wiederherstellung der Funktionalität der *Klura3-*20-bzw. *Klmet5-1-* Allele nach Integration erlauben. Die Antigen-codierende Sequenz wird über definierte Restriktionsschnittstellen zwischen die Promotor- und Terminatorsequenz der Expressionskassette kloniert. Mittels dieser Vektoren erfolgt die Integration von Fremdgen-exprimierenden Kassetten in das *K. lactis* Genom stabil, markerfrei und ohne Anwendung von Antibiotikaresistenzen. Die Stärken dieses Vektorsystems liegen entsprechend darin, dass Fremdgene einfach zwischen den verschiedenen Vektoren austauschbar sowie Promotoren und Terminatoren der Expressionskassetten durch andere ersetzbar sind. Die Expressionskassette besteht aus dem P*LAC4-12*-Promotor und dem *AgTEF1*-Terminator, sowie dem dazwischenliegenden Fremdgen. Das Fremdgen kann über die Restriktionsschnittstellen *Asc*I und *Not*I getauscht werden. Der P*LAC4*-*12*-Promotor kann in beiden Vektoren über die Restriktionsschnittstellen *Sma*I und *Asc*I ersetzt werden, der Terminator in *KI*pURA3 über *Not*I und *Box*l (bzw. *Mlu*I) und in *Kl*pMET5 über *Notl* und *Ecl*136II (bzw. *Sa*cl). Alternative Expressionskassetten werden in *KI*pURA3 zwischen die Restriktionsschnittstellen *Sma*I und *Box*I (oder *Mlu*I), in KIpMET5 zwischen *Sma*I und *Ecl*136II (oder *Sac*I) kloniert. Mit den genannten Restriktionsenzymen werden die Expressionskassetten auch zwischen *Kl*pMET5 und *KI*pURA3 Vektoren ausgetauscht oder zusätzliche Expressionskassetten eingebracht. Eine Verbesserung gegenüber dem *KI*p3- und *KI*p3-MCS-Vektoren (WO 20101054649) liegt darin, dass unter nicht-induzierenden Bedingungen (ohne Lactose) selektiert wird, was bei Proteinen mit CPE zu höheren Transformrationsraten führt und eine mögliche Anreicherung von Transformanten mit reduzierter Fremdgenexpression unterbindet. Siehe hierzu auch Beispiele 3.1 und 3.2.

In einer besonders bevorzugten Ausführungsform wird ein integrativer Expressionsvektor bereitgestellt, der ausgewählt ist aus *KI*pMET5-P*LAC4-12*-Et, *KI*pMET5-P*LAC4-12-LR2-*Et, *KI*pMET5-P*LAC4*-Et, *KI*pMET5*-PLAC4-LR2,* sowie aus *KI*pURA3-P*LAC4-12*-Et, *KI*pURA3-P*LAC4-12-LR2*-Et, *KI*pURA3-P*LAC4*-Et und *KI*pURA3-P*LAC4-LR2* (SEQ ID Nr.: 3 oder SEQ ID Nr. 4 in Kombination mit SEQ ID Nr.: 5, 6, 7 oder 8).

Die Vektoren *KI*pURA3-P*LAC4-12-*Et, *KI*pURA3-P*LAC4-12-LR2*-Et, *KI*pURA3-P*LAC4*-Et und *KI*pURA3-P*LAC4-LR2* sind Varianten des Vektors *KI*pURA3-Et, in die jeweils die codierende Nukleinsäuresequenz für das Etx.B-HA-Protein inseriert ist. Die Vektoren *KI*pURA3-P*LAC4-12*-Et, *KI*pURA3-P*LAC4-12-LR2*-Et, *KI*pURA3-P*LAC4*-Et und *KI*pURA3-P*LAC4-LR2* weisen Unterschiede im Promotor gegenüber dem Vektor *KI*pURA3-Et auf. Die Vektoren *KI*pMET5-P*LAC4-12*-Et, *KI*pMET5-P*LAC4-12-LR2*-Et, *KI*pMET5-P*LAC4-*Et, *KI*pMET5-P*LAC4-LR2* sind Varianten des Vektors *Kl*pMET5, in die jeweils die codierende Nukleinsäuresequenz für das Etx.B-HA- Protein inseriert ist. Die Vektoren *KI*pMET5-P*LAC4-12*-Et, *KI*pMET5-P*LAC4-12-LR2*-Et, *KI*pMET5-P*LAC4*-Et, *Kl*pMET5-P*LAC4-LR2* weisen Unterschiede im Promotor gegenüber dem Vektor *Kl*pMET5 auf.

Ein weiterer Aspekt betrifft ein Verfahren zur Herstellung eines *K*. *lactis*-Stammes gemäß der Erfindung, umfassend die Schritte:
(i) Insertion der codierenden Nukleinsäuresequenz des gewünschten Antigens in den *Kl*pURA3- oder *KI*pMET5-Vektor,
(ii) Transformation einer *K*. lactis-Kultur mit dem modifizierten und zuvor enzymatisch verdauten Vektorkonstrukt,
(iii) Selektion von transformierten *K. lactis-Zellen* mit Hilfe eines Festmediums, welches kein Uracil oder/und Methionin enthält, und
(iv) optional: Wiederherstellung der Prototrophie.

Gemäß einer Ausführungsform des Verfahrens können die Gensequenzen von mehreren Antigenen gleichzeitig ektopisch inseriert und reguliert exprimiert werden. Bevorzugt ist es, wenn unterschiedliche Gensequenzen ektopisch inseriert und reguliert exprimiert werden, die für Antigene verschiedener Varianten eines Pathogens codieren. Weiterhin bevorzugt ist es, wenn unterschiedliche Gensequenzen ektopisch inseriert und reguliert exprimiert werden, die für Antigene verschiedener Pathogene codieren.

In einem weiteren Aspekt stellt die Erfindung pharmazeutische bzw. veterinärmedizinische Zusammensetzungen für die parenterale, enterale, intramuskuläre, mukosale oder orale Verabreichung bereit, die einen *K. lactis* Stamm gemäß der Erfindung, optional in Kombination mit üblichen Träger- und/oder Hilfsstoffen enthält. Insbesondere betrifft die Erfindung pharmazeutische bzw. veterinärmedizinische Zusammensetzungen, die für die Vakzinierung geeignet sind.

Vorzugsweise umfasst die pharmazeutische bzw. veterinärmedizinische Zusammensetzung wenigstens einen physiologisch verträglichen Träger, Verdünnungsmittel, Adjuvans und/oder Hilfsstoff. Die *K. lactis* Stämme gemäß der vorliegenden Erfindung können in einem pharmazeutisch verträglichen Träger, z.B. in einem herkömmlichen Medium, wie einem wässrigen Salzmedium oder einer Pufferlösung als pharmazeutische Zusammensetzung zur Injektion enthalten sein. Ein solches Medium kann auch herkömmliche pharmazeutische Stoffe enthalten, wie zum Beispiel pharmazeutisch verträgliche Salze zur Einstellung des osmotischen Drucks, Puffer, Konservierungsmittel und dergleichen. Zu den bevorzugten Medien gehören physiologische Kochsalzlösung und Humanserum. Ein besonders bevorzugtes Medium ist PBS-gepufferte Kochsalzlösung.

Weitere geeignete pharmazeutisch verträgliche Träger sind dem Fachmann beispielsweise aus Remington's Practice of Pharmacy, 13. Ausgabe und J. of. Pharmaceutical Science & Technology, Vol. 52, Nr. 5, Sept-Okt., S. 238-311, bekannt.

Ein weiterer Aspekt der Erfindung betrifft die Verwendung der erfindungsgemäßen rekombinanten *K. lactis* Hefen zur Vakzinierung, wie beispielsweise zur Erzeugung einer protektiven Immunisierung, insbesondere einer protektiven Immunisierung, die gegen ein Pathogen gerichtet ist.

Ein entsprechendes Verfahren zur Erzeugung einer protektiven Immunisierung umfasst beispielsweise folgende Schritte:
a) Anzucht und Vermehrung der erfindungsgemäßen rekombinanten Hefen,
b) Ernte und Inaktivierung der Hefen,
c) Applikation der rekombinanten Hefen gemäß einem festzulegenden Immunisierungsschema,
d) Titerbestimmung der gebildeten Antikörper und/oder
e) Nachweis der Immunisierung.

Die Anzucht und Vermehrung der erfindungsgemäßen rekombinanten Hefen kann mit jedem konventionell verfügbaren Verfahren erfolgen. Besonders bevorzugt sind dabei Verfahren, die kostengünstig zu hohen Zellausbeuten führen. Dazu gehören Fermentationsverfahren, insbesondere Verfahren der Hochzelldichtefermentation. Als besonders vorteilhaft hat sich die Durchführung der Fermentation unter Anwendung eines Fed-Batch-Fermentationsprotokolls erwiesen.

In einer bevorzugten Ausführungsform wird die protektive Immunisierung dadurch erreicht, dass die rekombinanten Hefen oral/mukosal, intramuskulär oder subkutan appliziert werden.

Die rekombinanten Hefezellen sollten in dem erfindungsgemäßen Verfahren inaktiviert/abgetötet eingesetzt werden. Dazu werden die Hefe nach der Anzucht und Expression der Fremdgene getrocknet und anschließend inaktiviert. Die Inaktivierung kann mit jedem konventionell verfügbaren Verfahren durchgeführt werden. Besonders geeignet zur Anwendung in dem erfindungsgemäßen Verfahren sind die Hitzeinaktivierung (z.B. Hitzeinaktivierung für 2 Stunden bei 90°C) oder die γ-Bestrahlung (z.B. mit 25 oder 50 kGy).

Die Erfindung betrifft auch ein Verfahren zur Vakzinierung, umfassend die Verabreichung eines *K*. *lactis* Stammes gemäß der Erfindung an ein Subjekt, beispielsweise ein Tier oder einen Menschen, vorzugsweise ein Tier, in einer Menge, die ausreichend ist, um in dem Subjekt eine Immunantwort, vorzugsweise ein protektive Immunantwort gegen ein oder mehrere Fremdantigene auszulösen.

Ein besonderer Vorteil ist es, dass mit den *K. lactis* Stämmen gemäß der Erfindung bereits nach einer einmaligen Anwendung/Immunisierung ("*one shot*") oder nach einer zweifachen Anwendung/Immunisierung (*"prime-boost"*) eine protektive Immunantwort gegen ein Pathogen auslöst wird. Als weiterer Vorteil hat sich herausgestellt, dass mit den *K. lactis* Stämmen gemäß der Erfindung nach einer einmaligen Anwendung/Immunisierung ("*one shot*") oder nach einer zweifachen Anwendung/Immunisierung ("*prime-boost*") eine gegen verschiedene Varianten eines Pathogens kreuzprotektive Immunantwort ausgelöst werden kann. Wenn die *K. lactis* Stämme gemäß der Erfindung verschiedene Fremdgene gegen Antigene verschiedener Pathogene tragen und exprimieren, ist es sogar möglich, nach einer einmaligen Anwendung/Immunisierung ("*one shot*") oder in einer zweifachen Anwendung/Immunisierung (*"prime-boost"*) eine protektive Immunantwort gegen verschiedene Pathogene auszulösen.

### Zusammenfassung der Vorteile der Erfindung

Die beschriebenen Verbesserungen der *K*. *lactis* Plattform resultieren in zahlreichen Vorteilen:
a. Es wird eine starke Vereinfachung (***ready to use toolbox*/*****kit**)* und eine hohe Reproduzierbarkeit in der Stammkonstruktion von "*subunit-Vakzinen*" auf Hefebasis ermöglicht. Diese können nun in einer definierten, kurzen Zeitspanne erzeugt werden.
b. Die Hefevakzine können ein oder mehrere Antigene enthalten; diese können, flexibel angepasst, in unterschiedlichen Quantitäten produziert werden.
c. Zudem wird eine effiziente Fermentation der prototrophen Hefen ermöglicht.
d. Es wird eine strikte Induzierbarkeit der rekombinanten Proteinproduktion ermöglicht. Letztere ist besonders wichtig für Proteine, die einen CPE auslösen können.
e. Die gezielte, stabile, genomische Integration der Fremdgene und die damit einhergehende genetische Stabilität der Stämme bietet den Vorteil, dass Produktionsprozesse reproduzierbar ablaufen. Dies ist für die Produktion unter GMP besonders wichtig.
f. Mit der erreichten Steigerung der rekombinanten Antigenproduktion durch Erhöhung der Fremdgenkopien und/oder der KIGAL4-Konzentration wird die Protektivität des Hefevakzins verbessert.
g. Mit der erreichten Steigerung der rekombinanten Antigenproduktion durch Erhöhung der Fremdgenkopien und/oder der KIGAL4-Konzentration kann außerdem die zu applizierende Impfdosis reduziert werden. Die Hefeproduktion wird dadurch kosteneffizienter und die Verträglichkeit der Impfung für den Impfling verbessert.
h. Mehrvalente Hefevakzine können kreuzprotektiv oder mehrvalent protektiv zur Prophylaxe gegen verschiedene Varianten des gleichen Pathogens oder gegen unterschiedliche Pathogene eingesetzt werden. Abgesehen von der Inaktivierung und der Versetzung mit einem adäquaten Adjuvant bzw. einem geeigneten Flüssigkeitsvolumen ist kein weiteres *downstream processing* der Hefen für den Einsatz als Impfstoff nötig.

Die Erfindung wird nachfolgend anhand der Zeichnungen und Ausführungsbeispiele näher erläutert.

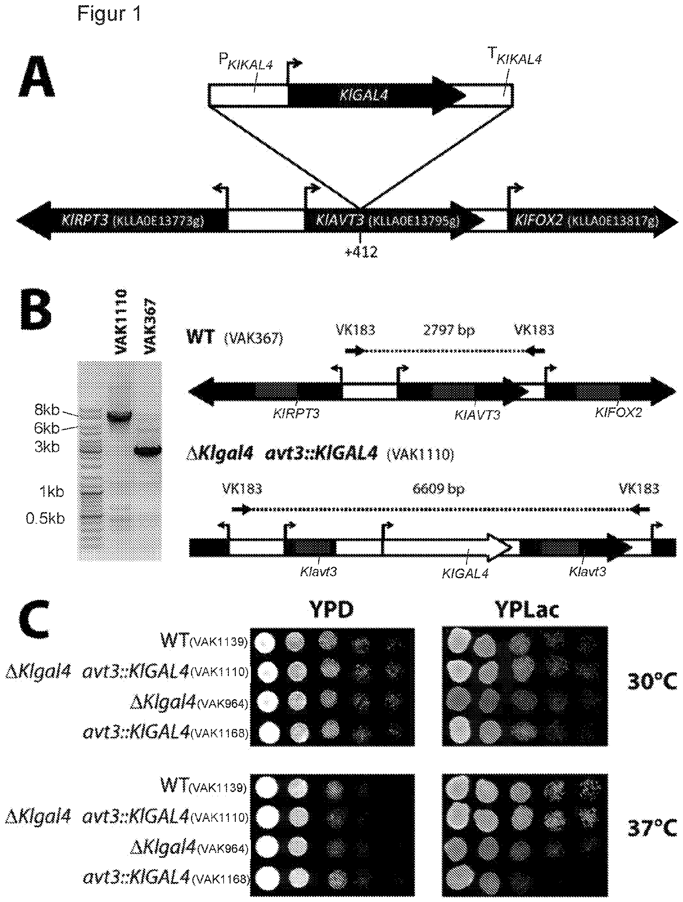

**Figur 1** zeigt die Charakterisierung eines neu generierten *K. lactis* Hintergrundstammes mit zwei *KlGAL4*-Kopien. Die Präsenz der zweiten ektopischen *KlGAL4-Kopie* am identifizierten Integrationsort wurde überprüft und der Effekt der Integration auf das Hefewachstum analysiert. **A:** Schema des Integrationsortes der ektopischen *KlGAL4*-Kopie. Der Integrationsort ist bezeichnet und die Gennamen sind gegeben. **B**: Agarosegel von mit den Primern VK183 (5'-GAGCCCACCACCTGCTCCTG-3') (SEQ ID Nr.: 9) und VK184 (5'-CTGATGTATTGCGCTCCTTACTAAC-3`) (SEQ ID Nr.: 10) PCR-amplifizierten Fragmenten des *KlAVT3*-Locus eines Hefestammes mit (VAK1110) und ohne (VAK367) zusätzlich integriertem, ektopischem *KlGAL4-Gen.* Die für den jeweiligen Fall erwarteten Fragmentgrößen sind im Schema rechts gegeben. **C:** Tropfentest mit seriellen zehnfach Verdünnungen (Start-OD 1) auf Glucose (YPD) oder Lactose (YPLac). Die Inkubation erfolgte jeweils bei 30°C und 37°C. Das Wachstum von Hefestämmen mit einer *KlGAL4*-Kopie am nativen Genlocus (VAK1139), am ektopischen Genlocus und deletiertem *KIGAL4* am nativen Genlocus (VAK1110), mit keiner KIGAL4.Kopie (Δ*KIgal4*; VAK964) oder mit zwei *KIGAL4*-Kopien (VAK1168), wurden verglichen. Es wird gezeigt, dass die definierte Integration eines weiteren *KlGAL4-Gens* lediglich zu marginalen Wachstumsdefekten führt: Diese sind lediglich bei 37°C und unter induzierenden Bedingungen sichtbar. Deutlicher ist der Wachstumsdefekt bei kompletter Deletion von *KlGAL4.*

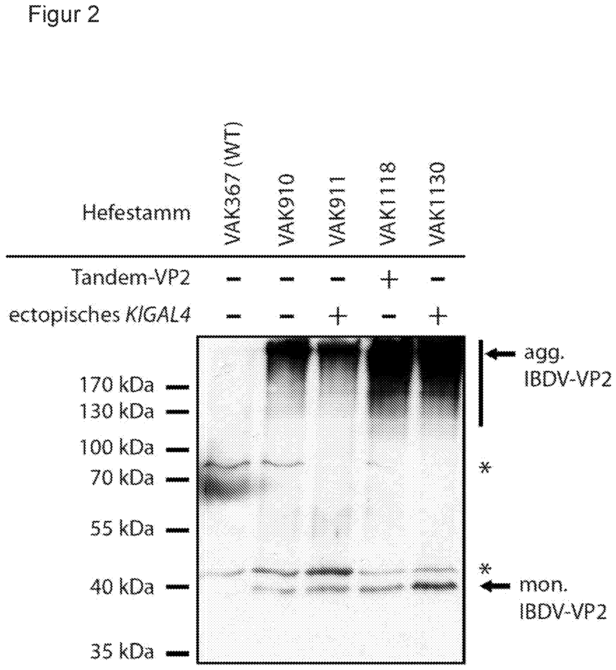

**Figur 2** zeigt die Westernblot-Analyse mit Proteinen eines IBDV-VP2-produzierenden *K. lactis* Stammes mit zusätzlicher, ektopischer *KlGAL4-Kopie.* Der Effekt einer zusätzlichen *KlGAL4*-Kopie auf die *LAC4*-12-Promotor-abhängige rekombinante Proteinproduktion, wurde per Westernblot analysiert. Als Teststamm diente ein Hefestamm mit einer IBDV-VP2-Expressionskassette der mit anderen IBDV-VP2-Hefestämmen verglichen wurde. Anwesenheit (+) oder Abwesenheit (-) einer ektopischen *KlGAL4*-Kopie und einer Tandem-IBDV-VP2-Expressionskassette (s.u.) sind oben angegeben. In Stamm VAK911 wurde die ektopische Kopie durch Linearisierung des Plasmides pLl-1 mittels *Bst*EII eingebracht (Krijger *et al.* 2012 und WO 2013107436), in Stamm VAK1130 war die ektopische *KIGAL4*-Kopie am *KlAVT3-*Locus (s. Fig. 1). Hefestamm VAK367 wurde als Wildtyp-Kontrolle ohne Fremdgen mitgeführt. Die Anzucht der Hefestämme erfolgte nach Vorkultur in YPD, während 15 h in YPLac. Jeweils 20 µg des Proteinextraktes wurden pro Hefestamm mittels SDS-PAGE analysiert. Der Immunoblot erfolgte mit anti-IBDV-Kaninchenserum (1:8000) und HRP-konjugiertem anti-Kaninchen-Antikörper aus Ziege (1:10.000). Multimeres (agg.) und monomeres (mon.) IBDV-VP2 sind rechts mit Pfeilen bezeichnet, unspezifische Banden mit Sternchen. Es wird gezeigt, dass die ektopische Expression eines zusätzlichen *KlGAL4-Gens,* ebenso wie die Präsenz einer Tandem-Expressionskassette (siehe auch unten) zu einer massiven Steigerung der Fremdantigenkonzentration führt.

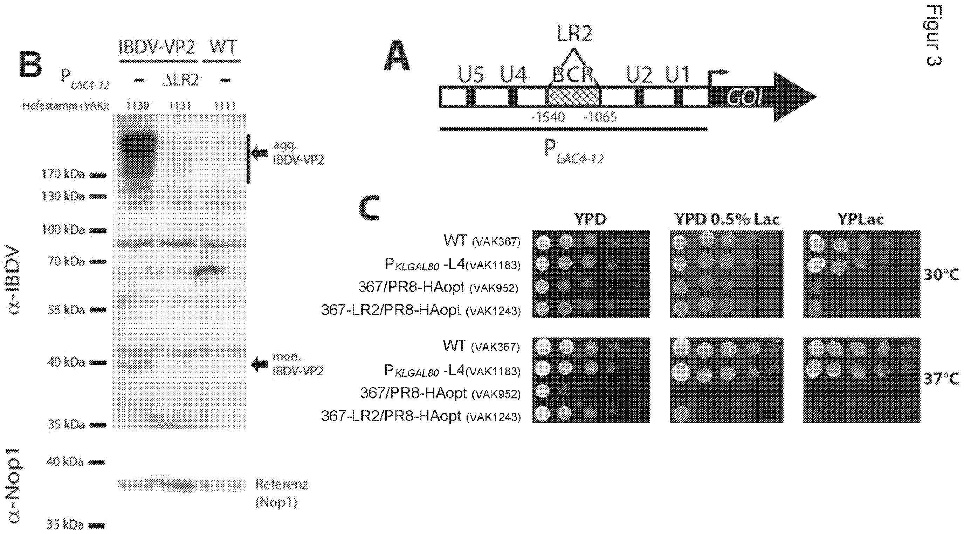

**Figur 3** veranschaulicht den Effekt der LR2-Deletion im *LAC4*-12-Promotor auf die nicht-induzierte, rekombinante Proteinproduktion und das Hefewachstum auf Glucose. Der nicht modifizierte *LAC4*-*12*-Promoter zeigt auch unter nicht induzierenden Bedingungen eine basale Expression des GOI (*gene of interest*). Dies ist besonders bei cytotoxisch wirkenden Fremdantigenen problematisch. Mit diesen Experimenten wurde getestet, ob durch eine Deletion in der BC-Region (LR2-Deletion) des *LAC4-12*-Promotors, die rekombinante Proteinproduktion unter nicht-induzierenden Bedingungen, reduziert oder sogar komplett unterdrückt werden kann. A: Schema eines *LAC4-12*-Promotors (P*LAC4-12*)*.* Die *basal control region* (BCR), die LR2-Deletion und die vier KIGal4-Bindestellen (*upstream activating sequence*: U1, U2, U4, U5) sowie die codierende Nukleinsäuresequenz des Fremdgenes (GOI) sind eingezeichnet. B: Westernblot von IBDV-VP2-Hefestämmen, mit (VAK1 131) und ohne (VAK1130) LR2-Deletion, nach Anzucht unter nicht-induzierenden Bedingungen (YP 3 % EtOH). VAK1111 diente als Wildtyp-Kontrolle ohne Fremdgen. Pro Hefestamm wurden 50 µg Proteinextrakt auf eine 12%iges-SDS-Gel geladen. Der Immunoblot erfolgte mit anti-IBDV-Kaninchenserum (1:5000) und HRP-konjugiertem anti-Kaninchen-Antikörper aus Ziege (1 :10.000). Die Ladekontrolle *KI*Nop1 wurde mit Maus anti-Nop1-Antikörper (1:5000) und HRP-konjugiertem anti-Maus-Antikörper aus Ziege (1 :10.000) nachgewiesen. C: Tropfentest mit seriellen zehnfach-Verdünnungen (Start-OD 1) auf YPD, YPD mit 0.5% Glucose und YPLac. Die Inkubation erfolgte jeweils bei 30°C und 37°C. Das Wachstum der am *LAC4*-Locus ein Influenza A HA-Fremdgentragenden Hefestämme, mit (VAK1243) und ohne (VAK952) LR2-Deletion, wurde verglichen. Als Wildtyp-Kontrollen ohne Fremdgen diente der Hefestamm VAK367. Es wird gezeigt, dass die LR2-Deletion die ungewollte, basale Fremdproteinexpression unterbindet. Ferner wird gezeigt, dass die LR2-Deletion das Wachstum eines Hefestammes, der ein cytotoxisches Protein (Influenza Hämagglutinin, HA) exprimiert sowohl unter nicht induzierenden, als auch unter induzierenden Bedingungen verbessert. Dies wird besonders deutlich bei 37°C.

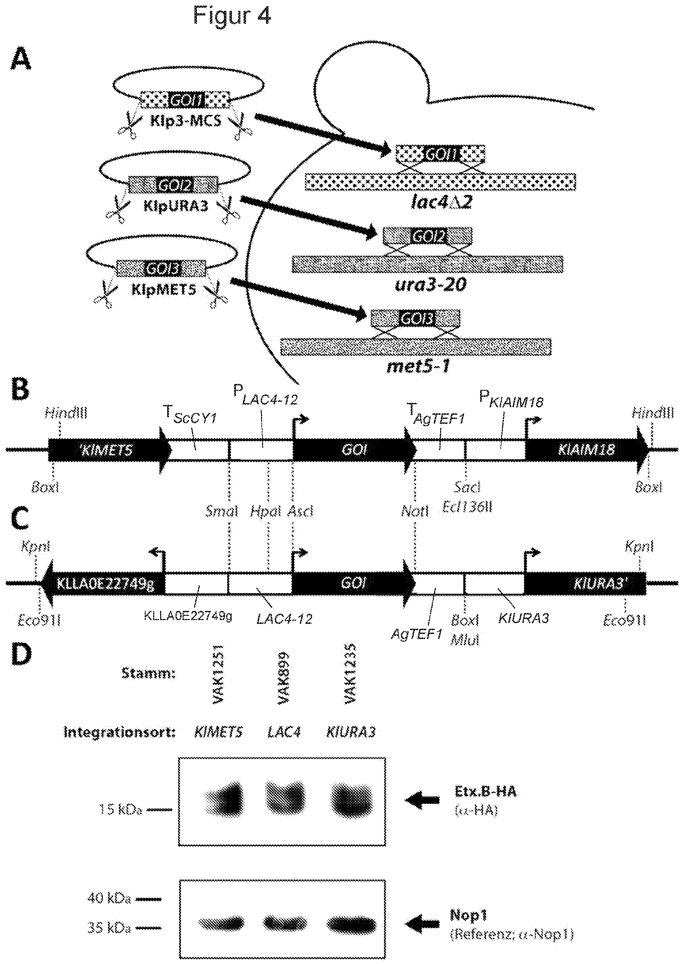

**Figur 4** zeigt die *Kl*p-Vektoren, die zur Integration von Proteinexpressionskassetten in verschiedene Loci des *K. lactis* Genoms genutzt werden können. Während die Nutzung des *LAC4*-Locus (*KI*p3-Vektorsystem) bereits beschrieben ist (WO 20101054649 und WO 2013107436), ist die Nutzung der *KlURA3-* und *KlMETS-Loci* neu. A: Schema der unterschiedlichen *Kl*p-Vektoren mit ihrem jeweiligen Integrationsort im Genom. B & C: Expressionskassetten und flankierende Enden in den hier neu beschriebenen *KI*pURA3 (B) und *KI*pMET5 (C) Vektoren. Die unterschiedlichen DNA-Sequenzabschnitte und relevanten Restriktionsschnittstellen sind bezeichnet. *GOI:* Fremdgen (*gene of interest*)*.* D: Westernblot-Analyse der Fremdproteinexpression in mit Hilfe der Klp Vektoren (A, B & C) konstruierten Hefestämme. Fremdgen ist hierbei Etx.B-HA. Das Hefe ,house keeping` KINop1 Protein (KLLA0C04389g) wurde als Ladekontrolle nachgewiesen. Die Hefestämme wurden nach Vorkultur in YPD (+U), 4h in YPLac (+U) angezogen. Pro Hefestamm wurden 30 µg Proteinextrakt auf eine 12% SDS-PAGE geladen. Der Immunoblot erfolgte mit monoklonalem Maus anti-HA (1:5000) und anti-KINop1 (1:5000; Santa Cruz, TX, U.S.A.) Antikörpern, sowie HRP-konjugiertem anti-Maus Antikörper aus Ziege (1 :10`000; Jackson ImmunoResearch, PA, U.S.A.). Es wird gezeigt, dass beide *KlURA3* und *KlMET5* Loci, in ähnlicher Weise wie der *LAC4* Locus (WO 20101054649 und WO 2013107436), für die heterologe Genexpression nutzbar sind.

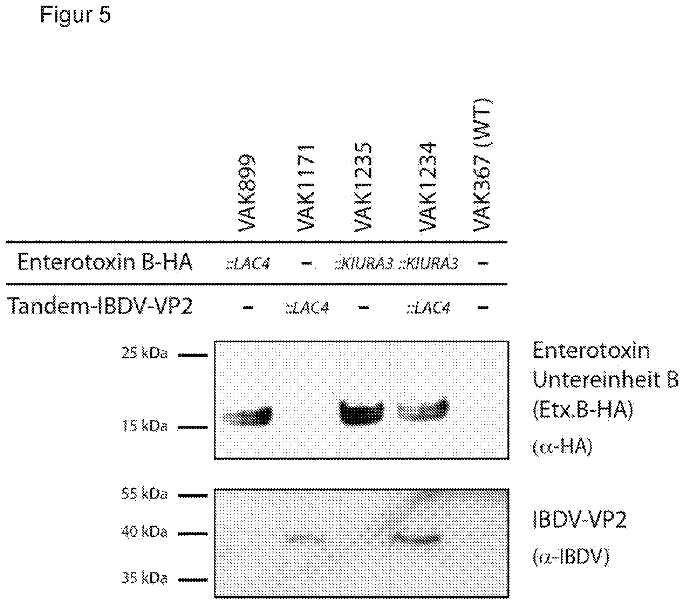

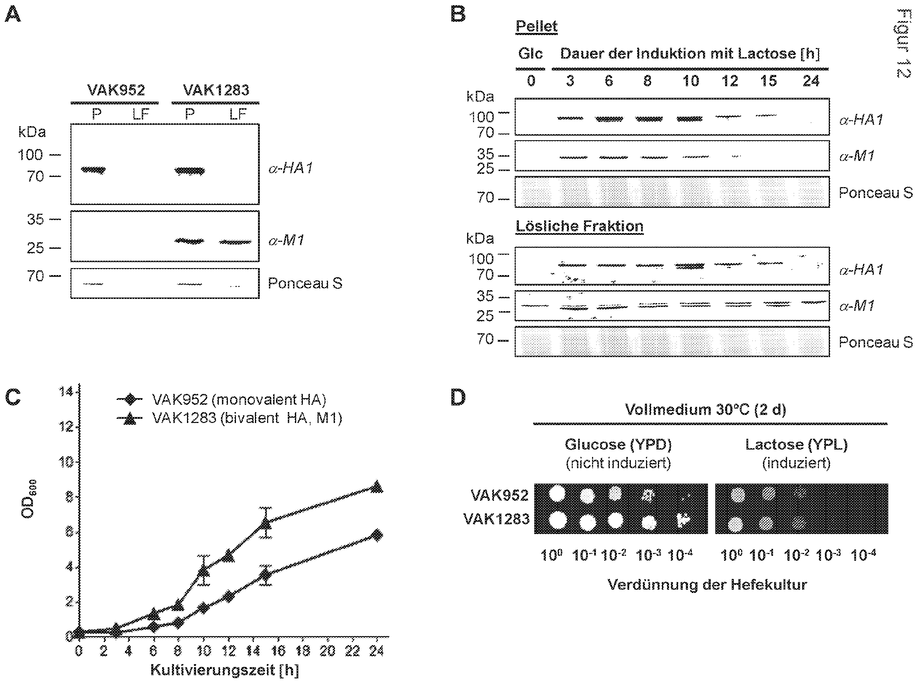

**Figur 5** zeigt die Produktion unterschiedlicher, rekombinanter Proteine im selben Hefestamm. Dieser Hefestamm (VAK1234) wurde mit den KIpURA3 und KIp3-MCS Vektoren konstruiert. Westernblot Analyse mit Proteinen eines Tandem-IBDV VP2-exprimierenden Hefestammes (s.u.), in den mit Hilfe des KIpURA3 Vektors eine zusätzlich Expressionskassette, mit Etx.B-HA als Fremdgen, eingebracht wurde (VAK1234). Als Kontrolle dienten Hefestämme die nur die Expressionskassette mit Etx.B-HA am *LAC4* (VAK899) bzw. *KlURA3* Locus (VAK1235) oder nur die Tandem-IBDV-VP2-Expressionskassette am *LAC4* Locus (VAK1 171) im Genom tragen. Nach Vorkultur in YPD wurden die Hefestämme über 6h in YPLac angezogen. Pro Hefestamm wurden 30 µg Proteinextrakt auf eine 12% SDS-PAGE geladen. Der Nachweis der Proteine im Immunoblot erfolgte für Etx.B-HA mit Maus anti-HA Antikörper (1 :5000; Santa Cruz, TX, U.S.A.) und HRP-konjugiertem anti-Maus Antikörper aus Ziege (1 :10`000) und für IBDV-VP2 mit Kaninchen anti-IBDV Antiserum (1 :5000; Granzow *et al.* (1997)) und HRP-konjugiertem anti-Kaninchen Antikörper aus Ziege (1 :10`000; Jackson ImmunoResearch, PA, U.S.A.). Es wird gezeigt, dass beide Fremdproteine in derselben Hefezelle exprimiert werden. Überraschenderweise wird das Expressionslevel eines Antigens, bei Ko-Expression eines anderen Antigens nicht eingeschränkt. Dies wird beim Vergleich der Expressionslevel in mono- und bivalenten Stämmen deutlich (siehe auch Fig. 12).

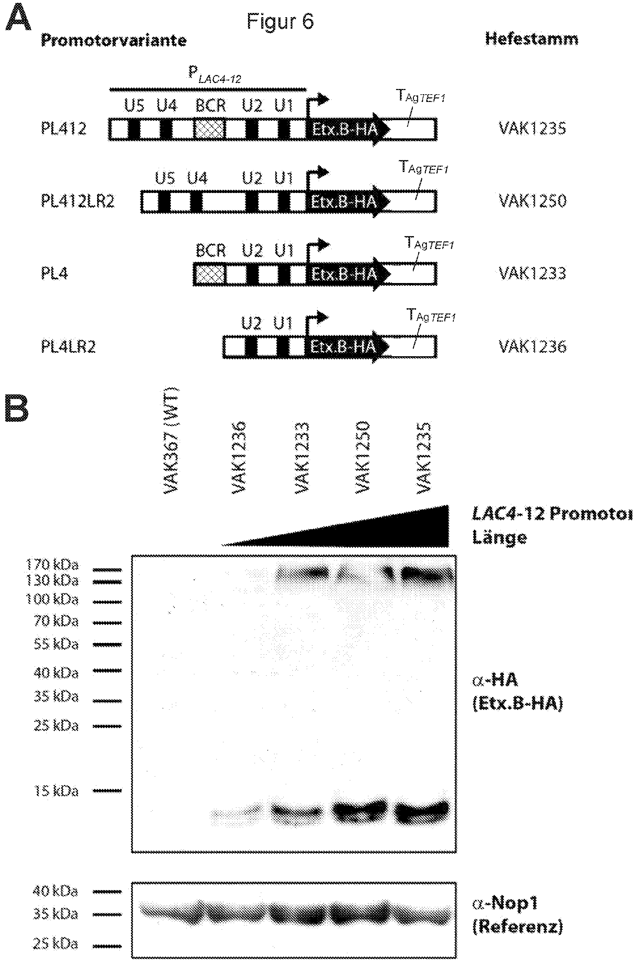

**Figur 6** zeigt die unterschiedlich stark induzierten *LAC4-12* Promotorvarianten für Expressionskassetten in KIp Vektoren. Die Expressionskassetten der KIp Vektoren wurden mit unterschiedlichen Varianten des *LAC4-12* Promotors versehen. Der Effekt der Promotorvarianten auf die Stärke der Induktion der Proteinsynthese, wurde getestet, anhand der Analyse von Hefestämmen, die die entsprechenden Expressionskassetten mit Etx.B-HA als Fremdgen enthalten. A: Schematische Darstellung der Promotorvariante, die dazugehörige KIpURA3 Vektoren mit Etx.B-HA als Fremdgen und die daraus erstellten Hefestämmen. BCR: Binderegion der Transkriptionsaktivatoren KICat8 und KISip4, Transkriptionsaktivatoren unter nicht-induzierenden Bedingungen; U1, U2, U4, U5: Binderegionen für den Transkriptionsaktivator KIGal4 (*upstream activating sequence*). B: Westernblot Analyse zur Charakterisierung der *LAC4-12* Promotorvarianten in den mit dem KIpURA3 Vektor erstellten Hefestämmen (A). Die Hefestämme wurden nach Vorkultur in YPD, 4h in YPLac angezogen. Pro Hefestamm wurden 30 µg Proteinextrakt auf eine 12% SDS-PAGE geladen. Der Immunoblot erfolgte mit monoklonalem Maus anti-HA (1:5000) und anti-Nop1 (1:5000) Antikörper, sowie HRP-konjugiertem anti-Maus Antikörper aus Ziege (1:10`000). Es wird gezeigt, dass die Expressionsrate des Fremdgens je nach Art des verwendeten Promoters unterschiedlich hoch ist.

**Figur 7** zeigt den Effekt der Verdoppelung der Anzahl der Fremdgenkopien mittels einer Tandem-Expressionskassette auf die rekombinante Proteinproduktion. Der Effekt auf die rekombinante Proteinproduktion (IBDV-VP2) durch Erhöhung der Anzahl Fremdgenkopien mittels einer Tandem-Expressionskassette wurde getestet. A: Schematische Darstellung der Tandem-Expressionskassette. DNA-Abschnitte und relevante Restriktionsschnittstellen sind bezeichnet. *GOI:* Fremdgen (*gene of interest*)*.* B: Das aus (A) abgeleitete Tandem Konstrukt zur zufälligen Integration mit Hilfe eines *ScURA3* Selektionsmarkers, ist dargestellt. C: Westernblot Analyse zum Vergleich der IBDV-VP2 Proteinproduktion in einem Hefestamm (VAK1118) mit einer Tandem-Expressionskassette (A) und einem Hefestamm (VAK910) mit einer Expressionskassette mit nur einer Fremdgenkopie. Die Hefestämme wurden nach Vorkultur in YPD, 3h, bzw. 6h in YPLac angezogen. Pro Hefestamm wurden 60 µg Proteinextrakt auf eine 12% SDS-PAGE geladen. Der Immunoblot erfolgte mit anti-IBDV Kaninchenserum (1:10'000) und HRP-konjugiertem anti-Kaninchen Antikörper aus Ziege (1:10'000). Aggregiertes (agg.) und monomeres (mon.) IBDV-VP2 sind rechts mit Pfeilen bezeichnet, unspezifische Banden mit Sternchen. D: Western Analyse von Hefestämmen mit zufällig integrierter Tandem-IBDV-VP2 Expressionskassette (B) im Vergleich zu einem mit KIp3-MCS erzeugtem Hefestamm mit einer Expressionskassette (VAK910) sowie dem daraus abgeleiteten mit zusätzlichen *KIGAL4-1* Kopien (pLI-1). Die Hefestämme wurden nach Vorkultur in YPD, 8h in YPLac angezogen. Der Immunoblot erfolgte wie unter (b) beschrieben. Es wird gezeigt, dass die Verwendung einer Tandem Expressionskassette die Fremdproteinexpressionsrate signifikant erhöht.

**Figur 8** zeigt die Genfragmente zur Wiederherstellung der Genfunktion der Allele *Klura3-20* und *Klmet5-1* (A). Schematisch sind die Genloci und die mit den angegebenen Primern amplifizierten Genfragmente für *KlURA3* (A) und *KlMET5* (B) dargestellt. Die Mutationen, der durch die homologe Rekombination mit diesen Genfragmenten rekonstituierten Allele *Klura3-20* (A) und *KImet5-1* (B) sind als Sternchen unterhalb der Gene gezeigt. Die Restriktionsschnittstellen, mit denen die subklonierten Fragmente ausgeschnitten werden, sind eingezeichnet. Dieses Schema verdeutlicht die Generierungsstrategie von prototrophen Fremdgen-exprimierenden Hefestämmen am *URA3* bzw. *MET5* Locus.

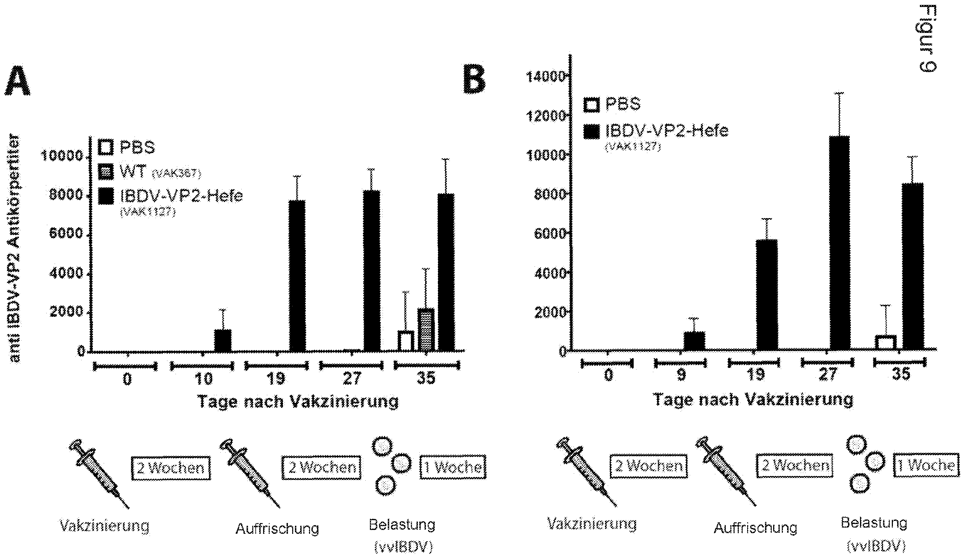

**Figur 9** veranschaulicht in Kombination mit Tab. 1 und Tab. 2 die protektive Immunisierung von Hühnern gegen vvIBDV in einem klassischen prime-boost Impfschema. In zwei Experimenten (A und B) wurden Gruppen von mindestens 16 SPF Hühnern subkutan nach einem *prime-boost* Verfahren mit lyophilisierten und hitze-inaktivierten Hefezellen des genetisch optimierten Tandem-IBDV-VP2 *K. lactis* Hefestammes VAK1127 geimpft. Die erste Impfung erfolgte zwei Wochen nach Schlupf (*prime*), die zweite (*boost*) zwei Wochen darauf. Zwei Wochen nach dem *boost* erfolgte ein Virus *challenge* mit einem hoch-virulenten (vv) IBDV-Stamm (*very virulent* 89163/7.3). Eine Probandengruppe, die als Infektionskontrolle diente, unterlief eine nachahmende (mock) Behandlung, bei der nur PBS bzw. Adjuvanz appliziert wurde. In Experiment 1 (A) wurde als Kontrolle auch die Wildtyphefe (VAK367) appliziert. Mindestens sieben Hühner pro Gruppe dienten als Kontrolle ohne Virus *challenge;* mindestens fünf in Experiment 2 (B). Seren wurden kurz vor der ersten Applikation gewonnen, vor und nach dem Challenge, ansonsten in zehn Tages Intervallen. Die Stärke der Serokonversation wurde mittels ELISA (ProFLOK IBD Plus, Synbiotics) bestimmt. Die nach Kit-Angaben umgerechneten Titer sind gezeigt. A: Experiment 2 erfolgte wie Experiment 1 (A). Der Mittelwert der ELISA-Titer von 12 Tieren ist mit Standardabweichung gezeigt. Beide Experimente zeigen eine massive Entwicklung von Titern von anti-IBDV VP2 Antikörpern bei den mit VAK1127 vakzinierten Tieren. Die assoziierten Tabellen fassen die Ergebnisse der Protektion der vakzinierten Tiere gegen *challenge* mit dem vvIBDV zusammen: In beiden Vakzinierungsexperimenten konnte eine vollständige Protektion gegen die virale Infektion erreicht werden.

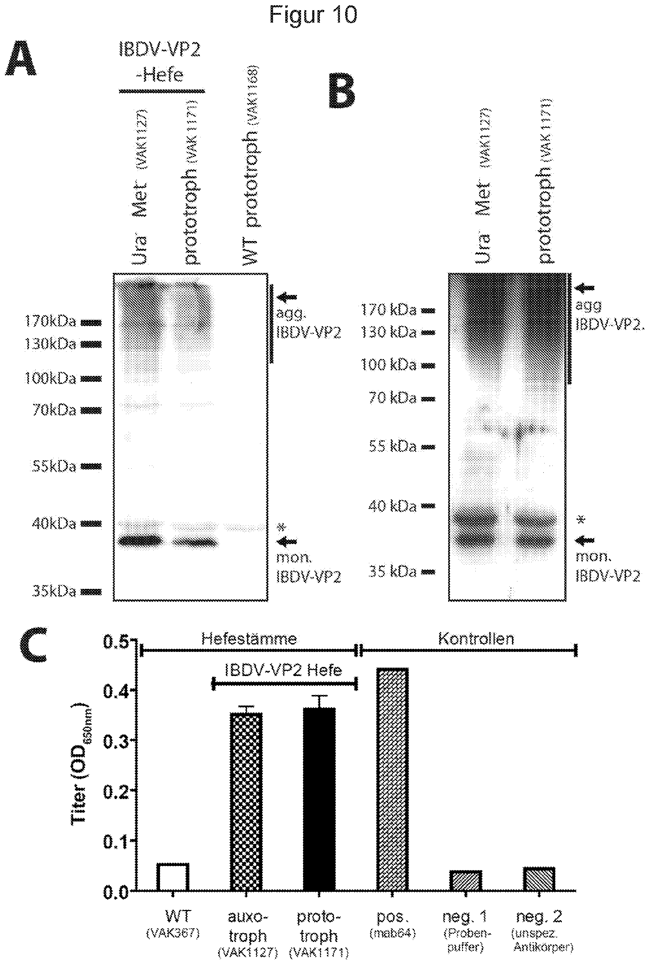

**Figur 10** zeigt den Effekt der genetischen Modifikationen zur Wiederherstellung der Prototrophie auf die rekombinante Proteinproduktionsmenge und Immunogenität eines Tandem-IBDV-VP2 Hefestammes. Der auxotrophe Tandem-IBDV-VP2-Hefestamm VAK1127 und der daraus abgeleitete prototrophe Hefestamm VAK1171 wurden bezüglich Effizienz der rekombinanten Proteinproduktion und Immunogenität verglichen. A: Westernblot Analyse zur Ermittlung des IBDV-VP2 Gehaltes in frisch geerntetem Hefematerial. Die Hefestämme wurden nach Vorkultur in YPD während 8h in YPLac angezogen. 40 µg Proteinextrakt je Hefestamm wurden auf eine 12% SDS-PAGE geladen. Der Immunoblot erfolgte mit anti-IBDV Kaninchen Antiserum (1:10`000) und HRP-konjugierten anti-Kaninchen Antikörpern aus Ziege (1:10'000). Aggregiertes (agg.) und monomeres (mon.) IBDV-VP2 sind rechts mit Pfeilen bezeichnet, unspezifische Banden mit Sternchen. B: Westernblot Analyse zur Ermittlung des IBDV-VP2 Gehaltes in lyophilisiertem, hitze-inaktiviertem Hefematerial, das danach in einer Immunisierungsstudie in BALB/c- Mäusen (C) eingesetzt wurde. Nach Vorkultur in YPD wurden die Hefestämme für 15h in YPLac angezogen. 10 µg Proteinextrakt wurden pro Hefestamm auf eine 12% SDS-PAGE geladen, ansonsten erfolgte der Immunoblot wie oben (A) und die Banden sind entsprechend bezeichnet. C: Test der Immunogenität der beiden Hefestämme VAK1127 und VAK1171 im Immunisierungsexperiment in BALB/c-Mäusen. Gruppen von jeweils fünf Mäusen wurden dreimalig subkutan geimpft mit 0.1 mg (Trockengewicht) des oben analysierten (B) Hefematerials. Als Kontrolle diente ein Wildtypstamm (VAK367) ohne Antigen. Die erste Applikation erfolgte mit CFA (*complete Freund's adjuvant*) als Adjuvant, die weiteren zwei, in zwei Wochen Intervallen, mit IFA (*incomplete Freund's adjuvant*) als Adjuvant. Eine Woche nach der dritten Applikation wurden die Mäuse euthanasiert und entblutet. Die Seren wurden per IBDV-VP2-ELISA (IDEXX) analysiert. Die Absorption bei 650 nm, korrelierend mit dem anti-IBDV-VP2 Antikörpertiter, ist mit Standardfehler gezeigt. Als Positivkontrolle für den ELISA diente ein monoklonaler anti-IBDV-VP2 Antikörper (pos. mab64), als Negativkontrolle wurde entweder Probenpuffer (neg. 1), oder ein unspezifischer Antikörper (neg. 2) eingesetzt. Es wird gezeigt, dass beide Stämme eine ähnliche Fremdproteinexpression und immunogenes Potenzial aufweisen.

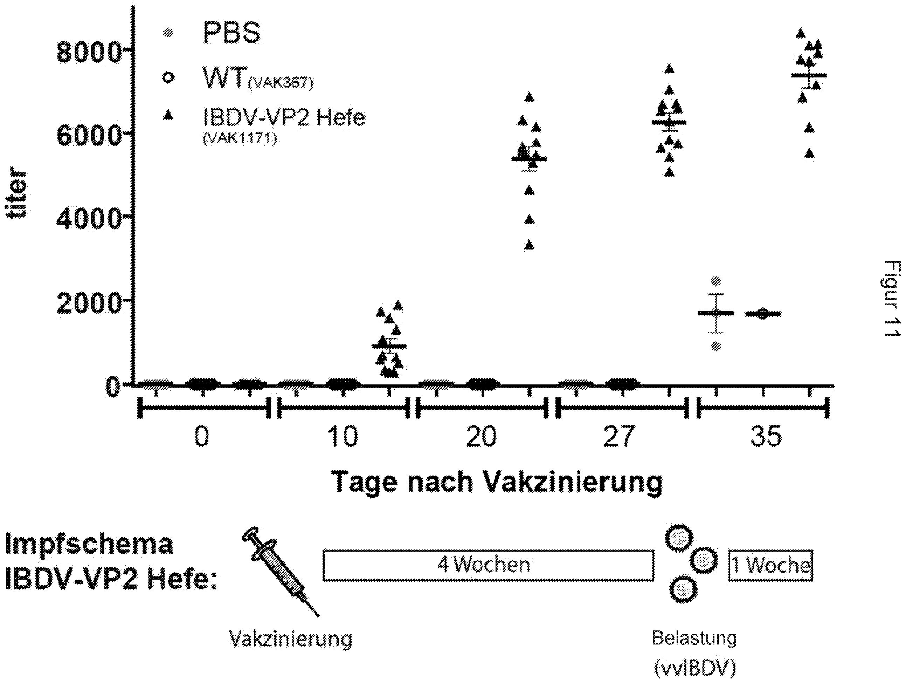

**Figur 11** zeigt in Kombination mit Tab. 3 die protektive Immunisierung von SPF Hühnern gegen vvIBDV durch einmalige, subkutane Applikation mit genetisch optimierter IBDV-VP2-Impfhefe. Gruppen von mindestens 18 SPF Hühnern wurden zwei Wochen nach Schlupf einmalig subkutan mit 10 mg hitzeinaktivierten Zellen des genetisch optimierten Tandem-IBDV-VP2 *K. lactis* Hefestammes VAK1171 geimpft. Als Kontrollen dienten mit PBS oder 10 mg VAK367 geimpfte Tiere. Diese wurden zweimalig geimpft, zwei, bzw. vier Wochen nach Schlupf. Der Challenge mit vvIBDV erfolgte bei allen Tieren sechs Wochen nach Schlupf. Die Seren wurden wie zuvor beschrieben per ELISA (ProFLOK IBD Plus, Synbiotics) analysiert. Die ermittelten Antikörpertiter sind gezeigt. Die einzelnen Punkte stellen individuelle Antikörpertiter der zwölf pro Gruppe analysierten Hühner dar, der Balken den Mittelwert mit Standardabweichung. Bei den Kontrollen wurde nach dem Challenge nur der Antikörpertiter der überlebenden Hühner ermittelt. Es wird gezeigt, dass bereits durch eine '*one shot* Vakzinierung mit dem Hefe *subunit* Vakzin VAK 1171, ein vollständiger Schutz gegen eine nachfolgende Belastung mit vvIBDV erreicht wird.

**Figur 12** zeigt die Charakterisierung der Stämme VAK952 und VAK1283. (A) Die Hefestämme VAK952 (monovalent HA) und VAK1283 (bivalent HA, M1) wurden im Schüttelkolben in YPD vorinkubiert und anschließend in YPL für 6 h induziert. Es wurde die optische Dichte bei 600 nm gemessen und 30 OD-Einheit der Kultur geerntet, das Pellet mit Glasperlen aufgeschlossen und die lösliche (LF) sowie die unlösliche (P, Pellet) Proteinfraktion im Immunoblot untersucht. Als Primärantikörper wurden α-HA1 oder α-M1 und als Sekundärantikörper α-mouse-IR-Dye800CW verwendet. Das Signal wurde über ein Infrarot-Imaging-System (LI-COR Biosciences) generiert. (B, C) Die Hefestämme wurden im Schüttelkolben in YPD vorinkubiert und anschließend über einen Zeitraum von 24 h in YPL induziert. Zu den angegebenen Zeitpunkten wurde die optische Dichte der Hefekultur bestimmt und 30 OD-Einheiten geerntet. (B) Die Pellets von VAK1283 wurden mit Glasperlen aufgeschlossen und im Immunoblot analysiert. (C) Die gemessenen Werte für die optische Dichte von VAK952 und VAK1283 wurde in Abhängigkeit von der Zeit als Wachstumskurve zusammengefasst und aus mindestens zwei voneinander unabhängigen Versuchen gemittelt. (D) Für den Tüpfeltest wurden die Hefestämme auf YPD-haltigen Nähragarplatten für 48 h bei 30°C angezogen. Beginnend mit 1 OD-Einheit wurden die Hefen seriell verdünnt und anschließend auf YPD- bzw. YPL-haltige Nähragarplatten getropft. Die Platten wurden für 48 h bei 30°C kultiviert und anschließend fotografiert. Ponceau S: Färbung Hefe-Totalprotein der jeweiligen Fraktion, Ladekontrolle. Es wird gezeigt, dass VAK952 (monovalent HA) und VAK1283 (bivalent (HA, M1) das HA Protein in vergleichbaren Quantitäten exprimieren. Ferner wird gezeigt, dass VAK1283 und VAK952 vergleichbare Wachstumseigenschaften aufweisen, mit leichten Vorteilen bei VAK1283.

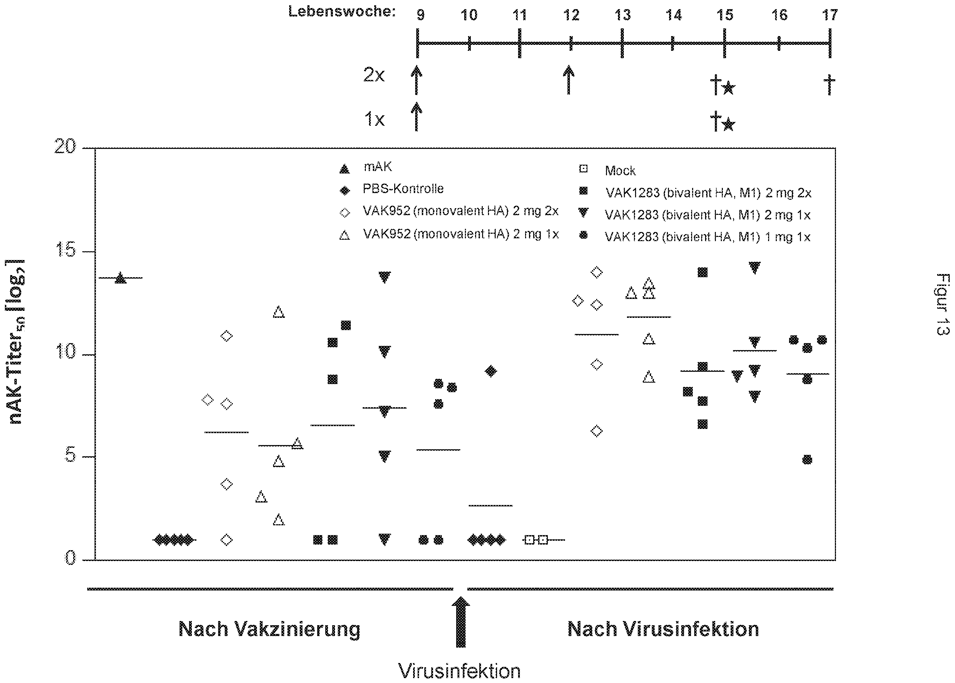

**Figur 13** veranschaulicht den Antikörpertiter im Serum von BALB/c Mäusen nach Immunisierung mit VAK952 (monovalent HA) sowie VAK1283 (bivalent HA, M1) vor und nach Belastungsinfektion. Beide Hefestämme wurden im Schüttelkolben mit YPD vorinkubiert und anschließend für 12 h (VAK952) bzw. 6 h (VAK1283) in YPL induziert. Anschließend wurden die Kulturen geerntet, gefriergetrocknet und das Hefematerial für 2 h bei 90°C inaktiviert. Für die Immunisierung wurden 9 Wochen alte, weibliche BALB/c Mäuse im Abstand von drei Wochen zweimal (*prime-boost*) oder einmal (*oneshot*) mit 2 mg Hefe (VAK952, VAK1283) bzw. mit 1 mg VAK1283 oder zweimal mit PBS (ohne Adjuvant) subkutan immunisiert. Als Adjuvant wurde AddaVax verwendet. Drei bzw. sechs Wochen nach der letzten Applikation wurden die Tiere mit der 5x MLD₅₀ des Influenza A/PR/8/34 (H1N1) Virus intranasal infiziert. Als Infektionskontrolle dienten scheininfizierte Tiere (Mock), denen nur PBS ohne Virus intranasal appliziert wurde. Drei bzw. sechs Wochen nach der letzten Applikation sowie während der Belastungsinfektion wurde das Serum der Tiere gewonnen und im VNT auf neutralisierende Antikörper (nAK) untersucht. nAK-Titer₅₀: Serumverdünnung, welche die Anzahl der Plaques im Vergleich zur Virus-freien Kontrolle noch um 50% reduziert. Angegeben ist der log₂ der entsprechenden Serumverdünnung. Aufgrund der logarithmischen Auftragung, wurde Serumproben ohne nachweisbare Antikörper der Wert: log₂(2)=1 zugeordnet. mAK: Testsystem-Kontrolle (α-H1 (H37-66)). Es wird gezeigt, dass beide Immunisierungsschemata zu einer signifikanten Induktion neutralisierende Ak führen. Ferner wird deutlich, dass sich die erhaltenen neutralisierenden Anti-HA Antikörpertiter bei den durchgeführten *prime-boost,* als auch bei den durchgeführten *one shot* Vakzinierungsexperimenten bei VAK952 und VAK1283 nicht signifikant unterscheiden.

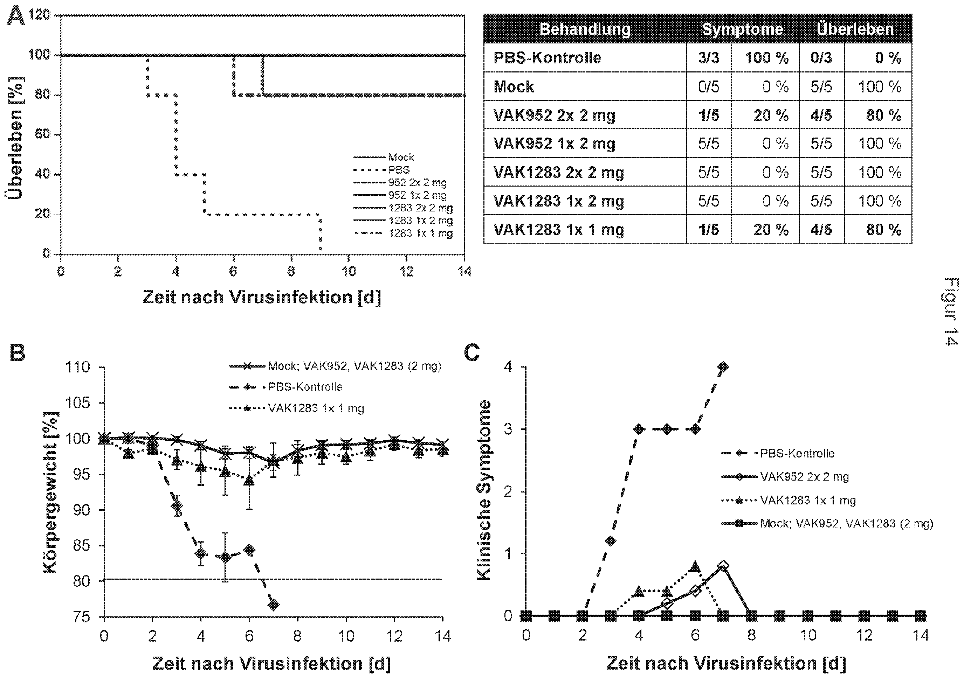

**Figur 14** zeigt die Belastungsinfektion mit Influenza A/PR/8/34 (H1N1) nach Immunisierung mit VAK952 (monovalent HA) sowie VAK1283 (bivalent HA, M1). Drei bzw. sechs Wochen nach der letzten Applikation (Immunisierungsschema siehe Fig. 13) wurden die BALB/c Mäuse mit der 5x MLD₅₀ des Influenza A/PR/8/34 (H1 N1) Virus intranasal infiziert. Als Infektionskontrolle dienten scheininfizierte Tiere (Mock), denen nur PBS ohne Virus intranasal appliziert wurde. Anschließend wurden das Überleben (A), das Gewicht (B) sowie klinische Symptome (C) der Tiere über einen Zeitraum von 14 Tage mehrfach täglich untersucht. Für die klinischen Symptome wurde ein Score von 0-4 festgelegt, welcher für jede Gruppe gemittelt wurde (0: Ohne Auffälligkeiten; 1: leicht struppiges Fell; 2: struppiges Fell, reduzierte Aktivität 3: struppiges Fell, Körpergewichtsverlust von 15%; 4: struppiges Fell, Körpergewichtsverlust von >20%). Es wird gezeigt, dass das prime-boost Immunisierungsverfahren mit VAK952 nicht optimal gegen eine Virusbelastung schützt, während dies bei VAK1283 der Fall ist. Das *one shot* Schema erzeugt mit beiden Vakzinen eine optimale Protektion mit 2 mg an verabreichtem Vakzin. Bei verabreichten 1 mg wird mit VAK1283 eine ähnliche Protektionsrate erreicht wie mit 2 mg VAK952 im prime-boost Verfahren.

### Ausführungsbeispiele

### Beispiel 1: Erzeugung eines Wirtsstamms mit zwei KIGAL4 Genkopien, stabil integriert, an nicht gekoppelten Genorten

Eine zweite *KlGAL4* Genkopie ohne Selektionsmarker wurde an einem anderen Genort (ektopisch) inseriert. Die Insertion konnte durch Sequenzierung im *KIAVT3* Gen *(KLLA0E13795g)* lokalisiert werden *(Klavt3::KIGAL4-1,* SEQ ID Nr.: 1) (Fig. 1). Der resultierende Stamm heißt VAK1111. Durch ein Kreuzungsexperiment wurde die unabhängige meiotische Segregation der beiden *KlGAL4* Kopien, die auf Chromosom E (ektopische Kopie) und D (genomische Kopie) liegen, bestätigt. Im selben Experiment wurde zudem die Anzahl von exakt zwei *KlGAL4-1* Genkopien im Genom festgestellt.

Zur Verwendung von VAK1111 für die gezielte Integration einer Expressionskassette am *LAC4* Locus analog zu VAK367-D4 wurde die *lac4::ScURA3* Disruption eingefügt, die erlaubt in einem Schritt, unter Selektion auf Lactose-Wachstum, das gewünschte Fremdgen mittels Klp-Vektortechnologie markerfrei zwischen *LAC4*-Promotor und *LAC4*-Leserahmen zu integrieren (Krijger et al. (2012)). Der resultierende Stamm VAK1123 unterscheidet sich von VAK367-D4 lediglich durch die zweite, ektopische *KlGAL4*-Genkopie.

### Beispiel 1.1: Verbesserte Produktivität eines Hefevakzinstammes mit zusätzlich integriertem KIGAL4 Gen.

In einem Anwendungsbeispiel wurde das IBDV-oVP2_{T2S} (Arnold et al. (2012)) Gen in den *LAC4*-Locus des Stammes VAK1123 inseriert (resultierender Stamm VAK1130). Es konnte eine erhöhte IBDV-VP2 Produktion gegenüber dem sonst isogenen Stamm mit nur einer *KlGAL4-Kopie* (VAK910), festgestellt werden. Als Vergleich ist zudem Stamm VAK1118 gezeigt, der nur ein *KlGAL4* Gen aber zwei *CDS VP2_{IBDV}* Kopien (siehe unten) trägt (Fig. 2).

### Beispiel 2: P_{LAC4-12LR2'} Promotor mit reduzierter basaler Aktivität zur Optimierung der Expression von Antigenen mit cytopathischem Effekt.

Die heterologe Proteinproduktion in Mikroorganismen ist dann problematisch, wenn dies zu einem cytopathischen Effekt (CPE) führt. Es stellte sich daher die Aufgabe, einen Weg zu finden, die Antigenproduktionsphase von der Biomasse-Akkumulationsphase zu entkoppeln. Durch den induzierbaren *LAC4*-Promotor ist dies durch einen *Fed-batch* Fermentationsprozess partiell möglich, wird aber erschwert, da der Promotor P*_{LAC4-12}* unter nicht-induzierenden Bedingungen nicht vollständig stillgelegt ist. Bei Antigenen mit sehr starkem CPE tritt eine Reduktion der Wachstumsrate und eine Induktion der zellulären Stressantwort auf, mit nachteiligen Effekten auf die Antigenproduktion. Durch die Verdoppelung der *KlGAL4*-Gendosis und/oder die Erhöhung der Anzahl Antigen codierenden Gene (s.u.) wird dieses Problem verschärft. Zur Lösung wurde die *basal control region* (BCR) des Promotors P*LAC4-12* (Fig. 3A) (Mehlgarten *et al.* (2015)) zwischen -1065 und -1540 deletiert (LR2 Deletion; P*LAC4-12-LR2_{'}; SEQ* ID Nr.: 2). Diese Deletion wurde in die Ausgangstämme VAK367 (eine *KIGAL4* Kopie) und VAK1111 (zwei *KIGAL4* Kopien) am genomischen *LAC4*-Locus gemeinsam mit der *lac4::ScURA3* Disruption eingeführt. Die resultierenden Stämme VAK1109 und VAK1124 sind geeignet für die Expression von Antigenen mit CPE. Der Promotor P*LAC4-12LR2'* wurde auch in die integrativen Vektoren KIpURA3-Et und KIpMET5-Et eingesetzt (s.u.).

### Beispiel 2.1:Inhibition der basalen (nicht induzierten) Antigenexpression durch modifizierten Promoter.

Nach Integration einer Tandem-IBDV-VP2 Expressionskassette in VAK1124 (resultierender Hefestamm: VAK1131; zur Erläuterung des Begriffs Tandem Expressionskassette`, s.u. und Fig. 7) konnte gezeigt werden, dass als Folge der LR2-Deletion im *LAC4*-12-Promotor die VP2 Proteinproduktion unter nicht induzierenden Bedingungen massiv reduziert ist (Fig. 3B). Mit Stämmen, die das Influenza A Antigen Hämagglutinin exprimieren (VAK952 ohne-, VAK1243 mit LR2-Deletion im Promoter) kann gezeigt werden, dass der cytopathische Effekt des Influenza A-HA Antigens unterdrückt und das Wachstum unter nicht-induzierenden Bedingungen durch die LR2-Deletion verbessert wird (Fig. 3C).

### Beispiel 3: Vielseitiges Vektorsystem für die gerichtete Integration von multiplen Expressionskassetten in das K. lactis Genom

Der Hefestamm VAK367 bildet, wie zuvor bereits für VAK367-D4 (Krijger *et al.* (2012), WO 20101054649), den genetischen Hintergrund aller hier beschriebenen *K. lactis* Stämme. Dieser Stammhintergrund weist durch Mutationen in zwei Genen, *KlURA3* (*KLLA0E22771g*) und *KIMET5 (KLLA0803938g),* die als Allele *Klura3-20* (fehlendes Basenpaar an Position +345) und *Klmet5-1* (G2555A; und A3682T) bezeichnet werden, eine Uracil- und Methionin-Bedürftigkeit (-Auxotrophie) auf; die Allele sind also nicht-funktionale Genvarianten.

Diese mutierten Allele wurden verwendet, um neben dem bereits mit dem KIp3/KIp3-MCS erschlossenen Integrationsort *LAC4* (Krijger *et al.* (2012)) weitere Loci für die gezielte Integration zu nutzen und dadurch multivalente Vakzinstämme zu generieren (Fig. 4A). Die Selektion erfolgt durch Wiederherstellung der Genfunktion dieser mutierten Gene ohne zusätzliche Insertion eines Selektionsmarkers. Dazu wurden neue Integrationsvektoren geschaffen. In diesen Vektoren werden die Expressionskassetten (jeweils unter Kontrolle des *LAC4*-12-Promotors oder dessen Varianten) durch Genabschnitte flankiert, die die Integration stromaufwärts des *KlURA3* Genes bzw. stromabwärts des *KlMET5* Gens durch homologe Rekombination erlauben und dabei die Wildtypsequenzen dieser Gene wiederherstellen.

Durch Mutagenese und Selektion auf Auxotrophie für alternative Wuchsstoffe lassen sich analog weitere Loci als Integrationsorte erschließen.

### Beispiel 3.1 :Vektoren KIpURA3 und KIpMET5 für die gerichtete Integration von Expressionskassetten (mit induzierbarem LAC4-12 Promotor) an den KIURA3 (KLLAOE22771g) und/oder KIMET5 (KLLAOB03938g) Loci von K. lactis Stämmen mit dem Klura3-20 bzw. KImet5-1 Allel.

Mittels geeigneter Genfragmente (*KIMET5*/*KlURA3 targeting sequences*), die eine gezielte Wiederherstellung der Funktionalität der *Klura3-20* bzw. *KImet5-1* Allele erlauben, wurden die integrativen Expressionsvektoren KIpURA3 (SEQ ID Nr.: 3) und KIpMET5 (SEQ ID Nr.: 4) konstruiert.

Der KIpMET5-Expressionsvektor enthält die Expressionskassette bestehend aus dem *LAC4*-12-Promotor (P*_{LAC4-12}* bzw. dessen Varianten), der codierenden Nukleinsäuresequenz des zu exprimierenden Antigens sowie dem *Ag*TEF1-Terminator; diese wird stromaufwärts vom genomischen *KI*MET5-Fragment mit eingebrachtem ScCYC1-Terminator und stromabwärts vom KIAIM18-Promoter mit nachgeschaltetem KIAIM18-Gen flankiert.

Der KIpURA3-Expressionsvektor enthält die Expressionskassette bestehend aus dem *LAC4*-12-Promotor (P*LAC4-12* bzw. dessen Varianten), der codierenden Nukleinsäuresequenz des zu exprimierenden Antigens sowie dem *Ag*TEF1-Terminator; diese wird stromaufwärts von KLLAOE22749g mit zugehörigem Promoter und stromabwärts vom KIURA3-Promoter mit nachgeschaltetem KIURA3-Fragment flankiert (Fig. 4B, C).

Die Antigen-codierende Sequenz wird jeweils über *Asc*I und *Notl* Schnittstellen zwischen Promotor und Terminator kloniert. Durch Restriktion mit *Eco*91I oder *Kpn*I des resultierenden Plasmids wird die gesamte Expressionskassette vom *KI*pURA3-, mit *Hind*III oder *Box*I vom KIpMET5-Vektorrückgrat getrennt und der Restriktionsansatz in *K. lactis* Wirtsstämme mit *Klura3-30* und/oder *KImet5-1* Allel transformiert. Die so in *KlURA3-20* bzw. *KlMET5-1* integrierte Expressionskassette mit Fremdgen entspricht also exakt jener, die auch in VAK367-D4 mit dem KIp3-MCS Vektor in *LAC4* integrierbar ist (WO 20101054649). Die Überprüfung Uracil- bzw. Methionin-prototropher Transformanten erfolgt standardmäßig über Kolonie-PCR mit den Primern MAB6 und VK211 für *KI*pMET5, bzw. den Primern MAB6 und VK71 für KIpURA3 Transformanten. Bei Integration der Expressionskassette am korrekten Zielort zwischen *KIURA3* bzw. *KIMET5* und dem jeweilig benachbarten Gen, ergeben sich Produkte mit der Grösse von 1652 bp für KIpMET5- bzw. 1307 bp für KIpURA3 Transformanten. Es wurden keine Hinweise erhalten, dass die Funktionalität der Nachbargene durch die Insertion beeinträchtigt ist.

### Primer:

**MAB6: 5**'-CCCAGATGCGAAGTTAAGTG-3' (SEQ ID Nr.: 11)
VK71: **5**'-TACAACAGATCACGTGATCTTTTTGTAAG-3' (SEQ ID Nr.: 12)
VK211: **5**'-GATTTCGTAACCCTATTGTTCATGAATG-3' (SEQ ID Nr.: 13)

### Beispiel 3.2: Expression eines Fremdantigens nach Integration der codierenden Genkassette am KIURA3 oder KIMET5 Locus.

Ein Fremdgen unter Kontrolle des P*_{LAC4-12}* Promotors wird nach Integration am *LAC4, KIURA3* und *KIMET5* Locus etwa gleich stark durch Lactose induziert. Die hitzelabile, nicht toxische, Untereinheit B des Enterotoxins (Etx.B) aus *E. coli* und einem (HA)₃-Epitop am C-Terminus (Etx.B-HA), diente als Testprotein zur Evaluierung des Vektorsystems. Die codierende Sequenz wurde in die Vektoren KIpMET5, KIpURA3 und KIp3-MCS kloniert und an den Genloci *KIMET5* (VAK1251), *KIURA3* (VAK1235) und *LAC4* (VAK899) integriert (Fig. 4D). Wie durch Western Blotting gezeigt, ist die Konzentration des Etx.B-HA Proteins in allen drei Stämmen sehr ähnlich (Fig. 4D). Demnach konnte kein Positionseffekt, abhängig vom Integrationsort der Expressionskassette im Genom, auf die rekombinante Proteinproduktionsmenge festgestellt werden.

### Beispiel 3.3: Ko-Expression zweier Fremdantigene in derselben Hefezelle.

Die Möglichkeit, über das neue Vektorsystem verschiedene heterologe Proteine unter Kontrolle des P*_{LAC4-12}* Promotors im gleichen Hefestamm zu produzieren, konnte durch die Konstruktion eines Hefestammes mit einer Etx.B-HA-Expressionskassette am *KIURA3*-Locus und einer Expressionskassette am *LAC4*-Locus mit zwei VP2_{IBDV} Kopien, die als Tandem vorlagen (VAK1234; Fig. 5; zur Erläuterung der Tandemkassette, siehe unten und Fig. 7), gezeigt werden. Gegenüber Hefestämmen, in denen jeweils nur eine der Expressionskassetten im Genom vorlag (VAK1235 bzw. VAK1171) konnte bei VAK1234 keine Reduktion der Proteinkonzentration von Etx.B-HA oder VP2_{IBDV} festgestellt werden.

### Beispiel 4: LAC4 Promotorvarianten zur Modulation der rekombinanten Proteinsynthese unter gleichartigen Induktionsbedingungen.

Die immunogene Wirkung von Antigenen beruht oft auf der Assemblierung mehrerer Proteine in nicht-stöchiometrischem Verhältnis. Um dies in hefebasierten Vakzinen zu ermöglichen, wurden Varianten des P*_{LAC4-**12LR2'**}* Promotors generiert (Fig. 6A), die sich durch Lactose oder Galactose unterschiedlich stark induzieren lassen. Sie sind gekennzeichnet durch die Anzahl an Bindestellen für den Aktivator KIGal4 (U1, U2, U4, U5; Gödecke et al. (1991)) und die An- bzw. Abwesenheit der basalen Kontrollregion BCR. Zusätzlich zu den in Fig. 3A gezeigten Konstrukten, die in den KIpURA3 Vektor eingesetzt wurden, können durch Insertion weiterer Bindestellen Promotor-Varianten mit erhöhter Promotorstärke erzeugt werden. Dadurch entstehen synthetische, Lactose-induzierbare Promotoren zur Erweiterung des Vektorsystems und es können unterschiedliche Proteinproduktions- bzw. Genexpressionsraten unter denselben Induktionsbedingungen realisiert werden.

### Beispiel 4.1: Expression eines Fremdantigens unter der Kontrolle verschiedener LAC4 Promotervarianten.

Expression von Etx.B-HA unter der Kontrolle von vier *LAC4-12* Promotorvarianten. Es wurden vier *LAC4* Promotorvarianten getestet, die sich in der Anzahl der Bindestellen für den Transkriptionsaktivator KIGal4 sowie der An-/Abwesenheit einer Kontrollregion für die basale Expression unter nicht induzierenden Bedingungen (*basal control region,* BCR; Fig. 6A; SEQ ID Nr.: 14) unterscheiden. Mit diesen wurden die Varianten des *KI*pURA3-Et Vektors *KI*pURA3-PL412-Et, *KI*pURA3-PL412LR2-Et, *KI*pURA3-PL4-Et und *KI*pURA3-PL4LR2 generiert und jeweils das Etx.B-HA Protein als Test-GOI inseriert. Die Insertion alternativer GOI ist, wie zuvor beschrieben, über die Schnittstellen *Asc*I und *Not*I möglich. Die Expressionskassetten wurden in den *KIURA3* Locus integriert und die Proteinkonzentration von Etx.B-HA über Western-Blotting quantifiziert (Fig. 6B). Es wird gezeigt, dass bei identischen Induktionsbedingungen (4h in Vollmedium mit Lactose) die längste Promotorvariante P*_{LAC4-12},* die die gesamte intergenische Region zwischen *LAC4* und *LAC12* Gen umfasst und vier KIGal4-Bindestellen (U1, U2, U4, U5) (Gödecke et al. (1991)) enthält, zur höchsten Proteinkonzentration führt. Wenn nur die zwei *LAC4* proximalen U1 und U2 Bindestellen vorhanden sind (-1064 bis -10), wirkt sich die zusätzliche Deletion der BCR (-1540 bis -1065) auch unter induzierenden Bedingungen proteinreduzierend aus.

### Beispiel 5: Steigerung der Antigen-Produktion durch Erhöhung der Kopienzahl des Antigen-codierenden Gens.

Es wurde daher das zuvor beschriebene Vektorsystem modifiziert, um schnell und effizient mehrere Genkopien hintereinander zu schalten und diese Expressionskassette in einem Schritt an einen der drei Genloci einzubringen (Fig. 7A). Zur Herstellung einer an den *LAC4*-Locus integrierbaren Tandem-Expressionskassette werden in einem Schritt von einer beliebigen KIp3(-MCS)-GOI Matrize drei PCR amplifizierte Fragmente fusioniert (*In-Fusion Cloning*): (1 und 2) Expressionskassette mit P_{LAC4-LR2} und T_{TEF} (Primer: VK30&VK31, bzw. VK32&VK33) und (3) LAC4 *targeting sequence* (VK34&VK35)). Nach Restriktion, z.B. mit *Hpa*I, lässt sich die Tandem-Expressionskassette, wie beschrieben, in den *lac4::URA3* Locus integrieren (Fig. 7). Nach erfolgreicher Integration der Expressionskassette wird die erste Fremdgenkopie je nach Ausgangstamm entweder durch oder P_{LAC4-12-LR2} reguliert und die zweite durch P_{LAC4-LR2}. Alternativ entsteht durch Insertion eines Selektionsmarkers zwischen den beiden Expressionskassetten in die Schnittstellen *Smi*I*, Mlu*I oder *Pme*I und Abtrennen der *LAC4 targeting sequence* über *Kpn*I, eine Tandem-Kassette, die über NHEJ ungerichtet in das Genom integriert werden kann. Wird die Expressionskassette mit *Mre*I und *Ava*I ausgeschnitten, können die kompatiblen Enden ligiert und dadurch lange, multiple Expressionskassetten generiert werden. Durch nochmalige Restriktion mit *Mre*I und *Ava*I werden im Ligationsgemisch Fragmente angereichert, bei denen die Expressionskassetten in Tandem (Kopf an Schwanz) angeordnet sind. Diese werden transformiert und unter Selektion auf den Marker ungerichtet integriert.

### Primer:

VK30:
   5'-
   TATAGGGCGAATTGGAGCTCCGCCGGCGGAAGAGGTAACGCCTTTTGTTAAC-3' (SEQ ID Nr.: 15)
VK31: 5'-CTAAACGGAACTCGCATTTAAATCTCGTTTTCGACACTGGATGG-3' (SEQ ID Nr.: 16)
VK32: 
5‘-
GCGAGTTCCGTTTAGACGCGTTTAAACTTGTTTAATTATTATGGGGCAGGCGAG
A-3‘ (SEQ ID Nr.: 17)
VK33: 5'-CGGGGAATGCGCTGCTTTTCGACACTGGATGGCGGCGTTA-3' (SEQ ID Nr.: 18)
**VK34: 5**'-GCAGCGCATTCCCCGGGTACCGCTCTCGACTAGGTGATTAGCG-**3**' (SEQ ID Nr.: 19)
VK35: **5**'-AAAAGCTGGGTACCGGGCCCACTAGTCGAGAGTTAACCGTGACTACAGCTA-**3**' (SEQ ID Nr.: 20)

### Beispiel 5.1: Erfolgreiche Anwendung der Multicopy-Strategie.

Die Strategie wurde mit IBDV-VP2 als Antigen und einer von KIp3 abgeleiteten Expressionskassette, die zwei IBDV-VP2 codierende Sequenzen (*CDS-VP2_{IBDV}*) in Tandem enthält, bestätigt. Die Tandem-IBDV-VP2-Expressionskassette (Fig. 7A) im KIp3 Vektor (Plasmid KIp3-Tandem-oVP2T2s,SEQ ID Nr.: 21) besteht aus zwei *LAC4* Promotor-regulierten codierenden Sequenzen für *VP2IBDV* (*CDS-VP2IBDV*) aus K/p3-MCS-oVP2T2s (Arnold *et al.*, (2012)). Die Promotorsequenzen bestehen aus der Region -1123 bis -10 des *LAC4*-Promotors für die erste und -1099 bis -10 für die zweite Kopie. Beide *CDS-VP2IBDV* sind am 3' Ende flankiert von einem *AgTEF1* Terminator. Das Plasmid *KI*p3-Tandem-oVP2T2s wurde mit *Hpa*I geschnitten und der Restriktionsansatz in Stamm VAK367-D4 transformiert. Der so erzeugte Hefestamm VAK1118 enthält die Tandem-Expressionskassette integriert am *LAC4*-Locus. Wie durch Western-Blotting gezeigt, ist eine höhere IBDV-VP2 Proteinkonzentration in diesem Stamm gegenüber dem isogenen Stamm mit nur einer Kopie vorhanden (Fig. 7B). Die Tandem-Expressionskassette ist genetisch sehr stabil: Nach Wachstum über 78 Generationen in induzierendem Medium (YNB + Lactose) zeigten unter 100 durch PCR getesteten Kolonien keine eine genetische Veränderung der Expressionskassette (Daten nicht gezeigt).

### Beispiel 6: Werkzeuge zur Herstellung einer Prototrophie in K. lactis Stämmen für die vereinfachte Fermentation in synthetischem Medium und Vollmedium.

In durchgeführten Studien hatte sich gezeigt, dass uracil-auxotrophe Hefestämme in Vollmedium schlechter wachsen als uracil-prototrophe Stämme, ein Effekt, der durch den Zusatz von Uracil nur teilweise aufgehoben werden konnte. Um die Fermentation der Vakzinstämme zu vereinfachen, die Etablierung der Produktionsprozesse zu erleichtern und kosteneffizienter zu machen und Wachstumseffekte durch ungenügende Aufnahme von Methionin und/oder Uracil zu vermeiden, sollten daher Wege gefunden werden, die Aufhebung dieser für die Stammkonstruktion notwendigen Auxotrophien schnell und reproduzierbar zu erreichen. Für die Rekonstitution von *KIURA3* aus *Klura3-20* wird mithilfe der Primer VK67 und VK69 und dem Wildtyp *KIURA3* Gen als Matrize ein DNA-Fragment über PCR generiert (**Fig**. **8A**). Zur Reparatur des *KImet5-1* Allels wird analog ein PCR Fragment mithilfe der Primer VK74 und VK75 und dem Wildtyp-Allel *KIMET5* als Vorlage generiert (**Fig. 8B**). Transformation der PCR Fragmente in die entsprechenden mutierten Stämme (einzeln oder gemeinsam) und Selektion auf Medium ohne Methionin und/oder ohne Uracil führte hoch effizient zur Rekonstitution der Wildtyp-Allele. Durchgeführt wurde dieser Prozess u.a. zur Generierung der Stämme VAK1171 und VAK1400 (s.o.).

### Primer

VK67: 5'-GACATCACTGTCTCTTCCCCTTAATGATC-3' (SEQ ID Nr.: 22)
VK69: 5'-TCAGCAAGCATCAATAATCCCCTTGGTTC-3' (SEQ ID Nr.: 23)
VK74: 5'-GAAAGAAAGACGTTGGTCTCTACGCTTG-3' (SEQ ID Nr.: 24)
VK75: 5`-AGATTATAAGTTCCTGGGGCTTTACCCAC-3` (SEQ ID Nr.: 25)

### Beispiel 7: Protektive Immunisierung durch optimierte, inaktivierte Vakzinhefen

Die gemäß den Beispielen 1 bis 5 erfolgten Modifikationen und Optimierungen der *K*. *lactis* Vakzinplattform wurden in verschiedenen Vakzinierungsstudien validiert.

### Beispiel 7.1: Immunogenität einer optimierten K. lactis Plattform, am Beispiel eines IBDV-VP2-Hefestammes (VAK1127).

Der VAK1127 Stamm enthält eine Tandem IBDV-VP2 Expressionskassette (SEQ ID Nr.: 21), zwei *KIGAL4* Kopien und die LR2 Deletion im *LAC4* Promotor. Zur Charakterisierung der Immunogenität des Hefestammes wurden Immunisierungsversuche im Ziel-Organismus Huhn durchgeführt. In *challenge* Experimenten wurde eine vollständige Protektion von SPF Hühnern gegen den von Eterradossi und Kollegen (1997) gut charakterisierten, hoch virulenten (vv) IBDV Stamm 89163/7.3 (AFSSA, Ploufragan, Frankreich) erreicht (Tab. 1 und 2). In den zwei unabhängig durchgeführten Versuchen wurde dazu zweimalig (Fig. 9A und B), subkutan 1 mg lyophilisierte, hitze-inaktivierte (2h, 90°C) Hefe (VAK1127) mit inkompletten Freund Adjuvanz (IFA) appliziert (prime-boost). Die Applikationen erfolgten zwei Wochen und vier Wochen nach Schlupf, die virale Belastung (*challenge*) sechs Wochen nach Schlupf. Nach 19 Tagen sind bei den mit VAK1127 vakzinierten Tieren bereits hohe Titer von anti-IBDV-VP2 Antikörpern messbar. In den Kontrollen treten Titer von anti-IBDV-VP2 Antikörpern erst nach *challenge* mit vvIBDV auf (Fig. 9). In beiden Experimenten wurde ein vollständiger Schutz (0% Morbidität, 0% Mortalität) der mit VAK1127 vakzinierten Tiere gegen den *challenge* mit vvIBDV beobachtet (Tab. 1 und 2). Mit diesen Experimenten konnte in einem klassischen *prime-boost* Impfverfahren ein Schutz gegen vvIBDV mit einem *subunit* Vakzin beobachtet werden.

Die Immunogenität der Impfhefen ist durch die genetische Rückmutation zu Antigentragenden prototrophen Hefestämmen nicht beeinflusst. Dies konnte mit Hilfe von den jeweils auxotrophen oder prototrophen Formen eines IBDV-VP2-Hefestammes in einem Vakzinierungsexperiment in Maus gezeigt werden (Fig. 10C). Der Hefestamm VAK1127 (auxotroph) wurde, wie oben beschrieben (Beispiel 6; Fig. 8), in zwei Schritten mit PCR-Fragmenten zur Erstellung von VAK1171 prototroph gemacht. Beide Stammformen weisen keinen signifikanten Unterschied im Expressionslevel von rekombinantem Protein auf (Fig. 10A und B). Die Mäuse wurden drei Mal subkutan in zwei Wochen Intervallen mit 0.1 mg hitze-inaktivierter Hefe subkutan mit IFA geimpft. Zwischen dem auxotrophen tBDV-VP2-Stamm (VAK1127) und dem prototrophen Abkömmling (VAK1171) konnte kein Unterschied in der Stärke der Serokonversation festgestellt werden (Fig. 10C).

### Beispiel 7.2: Vollständige Protektion durch Vakzinierung in einem ,one shot' Schema.

Eine ,one shot` Vakzinierung, d.h. Impfung durch einmalige Applikation des Vakzins, ist mit *subunit* Vakzinen normalerweise aufgrund mangelnder Immunogenität nicht wirksam. Die mit dem optimierten Stamm VAK1127 im *prime*/*boost* Verfahren erhaltenen Daten zur Entwicklung der Antikörpertiter (Fig. 9) deuteten jedoch auf die Möglichkeit hin, auch in einem *one shot* Ansatz eine Protektion zu erhalten. Um dies zu überprüfen, wurde eine *one shot* Vakzinierung mit dem prototrophen Hefestamm VAK1171 durchgeführt (Fig. 11; Tab. 3). Dazu wurde die Hefe nur einmalig appliziert, dafür in erhöhter Dosis (10mg), und es wurde dann im Abstand von 4 Wochen ein *challenge* durchgeführt. Es zeigte sich, dass mit VAK1171 mit '*one shot*' tatsächlich ein vollständiger Schutz gegen vvIBDV (0% Morbidität, 0% Mortalität) erreicht werden kann (Tab. 3). Zurückzuführen war dieses Ergebnis auf die Entwicklung hoher, protektiver Antikörpertiter, ca. 20 Tage nach Vakzinierung (Fig. 11). Die Tatsache, dass das *one shot* Impfschema hochprotektiv gegen vvIBDV schützt, zeigt das starke immunogene Potenzial des eingesetzten Vakzins und validiert beeindruckend die optimierte Vakzinplattform.

### Beispiel 7.3: Verbesserte Protektion eines bivalenten Hefevakzins gegenüber einem monovalenten Hefevakzins in der Anwendung gegen Influenza A Virus Infektionen.

Zur Vakzinierung gegen Influenzavirus Typ A wurden drei verschiedene Vakzinstämme generiert. Zum einen wurde VAK952 (DSM 32705) generiert, der das Hauptantigen eines Influenza A-Stammes (Puerto Rico/8/1934; PR8/34), das HA (Hämagglutinin) Gen exprimiert. Das Gen ist in VAK952 in der durch Krijger *et al.* (2012) und Arnold *et al.* (2012) beschriebenen Weise in den LAC4-Locus in das Genom integriert. Zum Zweiten wurde VAK1283 (DSM 32697) generiert. Hier ist zusätzlich zu dem HA-Gen aus PR8/34 im LAC4 Locus noch das M1 Gen in den URA3-Locus integriert. Das M1 Gen codiert für ein weiteres wichtiges Antigen von Influenza A, das deutlich konservierter ist als das HA. in bereits veröffentlichten Berichten konnte gezeigt werden, dass durch Kombination beider Antigene die Immunogenität eines Vakzins gegen Influenza A gesteigert und auch eine Kreuzprotektivität gegen verschiedene Influenzaviren erreicht werden kann. Um auch diesen Aspekt mit einem bivalenten Hefevakzin zu validieren, wurde ein weiterer Stamm (VAK1395; DSM 32706) generiert, der ebenfalls das M1 Gen im URA3 Locus enthält und wo das HA-Gen aus PR8/34 durch das HA-Gen des Influenzavirus California/4/2009 ersetzt ist. Die vergleichbare Expression von HA bzw. die zusätzliche Expression von M1 der jeweiligen Stämme wurde überprüft; ebenso wurde gezeigt, dass die Stämme ein vergleichbares Wachstum aufweisen, mit leichten Vorteilen bei VAK1283 gegenüber VAK952 (Fig. 12). In Vakzinierungsstudien, in denen jeweils ein *prime-boost* bzw. *one shot* Schema mit unterschiedlichen Hefekonzentrationen im Mausmodell angewendet wurden, wurde gezeigt, dass VAK952 und VAK1283 jeweils vergleichbare Titer Virus-neutralisierender Antikörper induzieren (Fig. 13). Im *challenge* Experiment wurde dann aber deutlich, dass das bivalente VAK1283 Vakzin sowohl im *prime-boost* Schema, als auch im *one shot* Schema eine maximale Protektion ermöglicht, während dies mit dem monovalenten VAK952 Vakzin nicht der Fall ist. Zudem wurde mit dem Vakzin VAK1283 im one shot Experiment bei der Hälfte des eingesetzten Hefematerials ein ähnlicher protektiver Effekt erreicht, wie mit VAK952 im *prime-boost* Ansatz (Fig. 14 und Tab. 3). In Experimenten, in denen VAK1395 als Vakzin eingesetzt wurde, konnte auch eine Protektion gegen Influenza PR8/34 festgestellt werden. Damit wurde mit einem bivalenten Hefevakzin eine Kreuzprotektion gegen verschiedene Influenzavarianten erreicht.

**Tabelle 1**

| **Indikationen für eine *Belsatungs* - Protektion in vakzinierten SPF Hühnern** | | | | | | | | | | | |
|---|---|---|---|---|---|---|---|---|---|---|---|
| **Impfung (a)** | | | **Histopathologische Bursaläsions Bewertung** | | | | | **bu/bod index (c)** | | | |
| **Hefestamm (VAK)** | **VP2 Menge per Impfdosis** | **Adjuvanz** | **0** | **1** | **2** | **3** | **4** | **belastet** | **unbelastet** | **Morbidität (%) (d)** | **Mortalität (%) (e)** |
| 367 | kein | IFA | - | - | - | 1 | 7 | 2.80 ± 1.32 | 5.36 ± 0.65 | 6/10 (60) | 4/10 (40) |
| 1127 | 4.1 ± 0.25 µg | IFA | 8 | - | - | 1 | - | 4.40 ± 0.76 | 4.89 ± 0.63 | 0/10 | 0/10 |
| - | PBS | IFA | - | - | - | - | 10 | 4.08 ± 1.91 | 4.92 ± 0.94 | 10/10 (100) | 8/10 (80) |

**Tabelle 2**

| **Indikationen für eine *Belastungs* - Protektion in vakzinierten SPF Hühnern** | | | | | | | | | | | |
|---|---|---|---|---|---|---|---|---|---|---|---|
| | | | | | | | | | | | |

| **Impfung (a)** | | | **Histopathologische Bursaläsions Bewertung** | | | | | **bu/bod index (c)** | | | |
|---|---|---|---|---|---|---|---|---|---|---|---|
| **Hefestamm (VAK)** | **VP2 Menge per Impfdosis** | **Adjuvanz** | **0** | **1** | **2** | **3** | **4** | **belastet** | **unbelastet** | **Morbidität (%) (d)** | **Mortalität (%) (e)** |
| 1127 | 4.1 ± 0.71 µg | IFA | 6 | - | - | - | - | 5.10 ± 0.78 | 4.81 ± 1.20 | 0/9 (0) | 0/9 (0) |
| - | PBS | IFA | - | - | - | - | 8 | 4.09 ± 1.87 | 5.32 ± 0.85 | 9/9 (100) | 7/9 (78) |

**Tabelle 3**

| **Indikationen für eine *Belastungs* - Protektion in vakzinierten SPF Hühnern** | | | | | | | | | | | |
|---|---|---|---|---|---|---|---|---|---|---|---|
| **Impfung (a)** | | | **Histopathologische Bursaläsions Bewertung** | | | | | **bu/bod index (c)** | | | |
| **Hefestamm (VAK)** | **VP2 Menge per Impfdosis** | **Adjuvanz** | **0** | **1** | **2** | **3** | **4** | **belastet** | **unbelastet** | **Morbidität (%) (d)** | **Mortalität (%) (e)** |
| PBS | kein | MF59 | - | - | - | - | 9 | 3.73 ± 1.92 | 4.77 ± 1.02 | 9/9 (100) | 6/9 (66) |
| VAK367 | kein | MF59 | - | - | - | - | 10 | 4.09 ± 1.58 | 3.60 ± 0.89 | 10/10 (100) | 9/10 (90) |
| VAK1171 | 35 ± 4.2 µg | IFA | 10 | - | - | - | - | 4.48 ± 0.37 | 3.96 ± 1.02 | 0/10 (0) | 0/10 (0) |

### Erläuterungen zu Tabelle 1

(a) Die Hühnerwurden zwei Wochen nach Schlupf subkutan mit 1 mg Hefe (oder PBS) und IFA als Adjuvanz geimpft. Zwei Wochen nach der Impfung wurden sie auf dieselbe Weise *geboostet.* Weitere zwei Wochen später erfolgte der virale Belastungstest *via* der ocunasalen Route mit 10⁴ EtD vvIBDV (very virulent 89163/7.3). Inaktivierte, ganze Hefe des Stammes VAK1127 wurde als Impfhefe verwendet, als Infektionskontrolle diente eine Gruppe, die nur mit PBS und IFA geimpft wurde. Als Kontrolle für den Effekt der Hefe allein fungierte eine Gruppe, in der wildtyp Hefe ohne Antigen (VAK367) appliziert wurde.
(b) Die histopathologische Bursaläsions Bewertung erfolgte mit einer Skala von 0-4: 0: keine Läsionen; 1: 5-25% der Folikel betroffen; 2: 26-50% der Folikel betroffen; 3: 51-75% der Folikel betroffen; 76-100% Bursaschädigung (Strukturverlust)
(c) Der Mittelwert der des Bursa zu Körpergewichts Indexes (bu/bod) wurde berechnet mit der Formel: (Bursagewicht/Körpergewicht)*1000. Die unbelastete Kontrollgruppe bestand aus mindestens sieben Hühnern, die belastete Gruppe aus zehn. Die Standardabweichung ist gegeben.
(d) Die Morbidität ist dargestellt als Anzahl morbider Hühner pro Anzahl Hühner der Gruppe insgesamt. Die Prozentzahl morbider Hühner ist in Klammern gezeigt.
(e) Die Mortalität ist dargestellt als Anzahl gestorbener Hühner pro Anzahl Hühner der Gruppe insgesamt. Die Prozentzahl verstorbener Hühner ist in Klammern gezeigt.

### Erläuterungen zu Tabelle 2

(a) Die Hühnerwurden zwei Wochen nach Schlupf subkutan mit 1 mg Hefe (oder PBS) und IFA als Adjuvanz geimpft. Zwei Wochen nach der Impfung wurden sie auf dieselbe Weise *geboostet.* Weitere zwei Wochen später erfolgte der virale Belastungstest *via* der ocunasalen Route mit 10⁴ EtD vvIBDV (very virulent 89163/7.3). Inaktivierte, ganze Hefe des Stammes VAK1127 wurde als Impfhefe verwendet, als Infektionskontrolle diente eine Gruppe, die nur mit PBS und IFA geimpft wurde.
(b) Die histopathologische Bursaläsions-Bewertung erfolgte mit einer Skala von 0-4: 0: keine Läsionen; 1: 5-25% der Folikel betroffen; 2: 26-50% der Folikel betroffen; 3: 51-75% der Folikel betroffen; 76-100% Bursaschädigung (Strukturverlust).
(c) Der Mittelwert des Bursa- zu Körpergewichts-Indexes (bu/bod) wurde berechnet mit der Formel: (Bursagewicht/Körpergewicht)*1000. Die unbelastete Kontrollgruppe bestand aus mindestens fünf Hühnern, die belastete Gruppe aus neun. Die Standardabweichung ist gegeben.
(d) Die Morbidität ist dargestellt als Anzahl morbider Hühner pro Anzahl Hühner der Gruppe insgesamt. Die Prozentzahl morbider Hühner ist in Klammern gezeigt.
(e) Die Mortalität ist dargestellt als Anzahl gestorbener Hühner pro Anzahl Hühner der Gruppe insgesamt. Die Prozentzahl verstorbener Hühner ist in Klammern gezeigt.

### Erläuterungen zu Tabelle 3

(a) Die Hühner wurden zwei Wochen nach Schlupf subkutan mit 10 mg Hefe (oder PBS) und IFA als Adjuvanz geimpft. Vier Wochen später erfolgte der virale Belastungstest *via* der ocunasalen Route mit 10⁴ EtD vvIBDV (very virulent 89163/7.3). Inaktivierte, ganze Hefe des Stammes VAK1171 wurde einmalig als Impfhefe verwendet. Als Infektionskontrolle diente einerseits eine Gruppe, die nur mit PBS und MF59, sowie eine Gruppe, die mit wildtyp Hefe und MF59 geimpft wurde, beiden wurde zwei Wochen nach der ersten Impfung ein *boost* mit derselben Menge an Hefe, bzw. PBS verabreicht.
(b) Die histopathologische Bursaläsions-Bewertung erfolgte mit einer Skala von 0-4: 0: keine Läsionen; 1: 5-25% der Folikel betroffen; 2: 26-50% der Folikel betroffen; 3: 51-75% der Folikel betroffen; 76-100% Bursaschädigung (Strukturverlust).
(c) Der Mittelwert des Bursa- zu Körpergewichts-Indexes (bu/bod) wurde berechnet mit der Formel: (Bursagewicht/Körpergewicht)*1000. Jede Gruppe bestand aus mindestens neun Hühnern. Die Standardabweichung ist gegeben.
(d) Die Morbidität ist dargestellt als Anzahl morbider Hühner pro Anzahl Hühner der Gruppe insgesamt. Die Prozentzahl morbider Hühner ist in Klammern gezeigt.
(e) Die Mortalität ist dargestellt als Anzahl gestorbener Hühner pro Anzahl Hühner der Gruppe insgesamt. Die Prozentzahl verstorbener Hühner ist in Klammern gezeigt.

### Sequenzen

Die Patentanmeldung enthält folgende Sequenzen als Bestandteil der Beschreibung:

| **SEQ ID. Nr.** | **Bezeichnung** |
|---|---|
| 1 | *K. lactis avt3*::*LAC9* |
| 2 | P*LAC4-12-LR2* |
| 3 | *KI*pURA3-Vektor |
| 4 | *KI*pMET5-Vektor |
| 5 | *LAC4*-12-Promotorvariante PLAC4-12 |
| 6 | LAC4-12-Promotorvariante P*LAC4-12-LR2* |
| 7 | LAC4-12-Promotorvariante P*LAC4* |
| 8 | LAC4-12-Promotorvariante P*LAC4-LR2* |
| 9 | Primersequenz VK183 |
| 10 | Primersequenz VK184 |
| 11 | Primersequenz MAB6 |
| 12 | Primersequenz VK71 |
| 13 | Primersequenz VK211 |
| 14 | BCR aus P*LAC4-12* |
| 15 | Primersequenz VK30 |
| 16 | Primersequenz VK31 |
| 17 | Primersequenz VK32 |
| 18 | Primersequenz VK33 |
| 19 | Primersequenz VK34 |
| 20 | Primersequenz VK35 |
| 21 | *KI*p3-Tandem-oVP2T2S |
| 22 | Primersequenz VK67 |
| 23 | Primersequenz VK69 |
| 24 | Primersequenz VK74 |
| 25 | Primersequenz VK75 |

### Referenzen

Arnold, M.; Durairaj, V.; Mundt, E.; Schulze, K.; Breunig, K. D. & Behrens, S.-E. Protective Vaccination against Infectious Bursal Disease Virus with Whole Recombinant Kluyveromyces lactis Yeast Expressing the Viral VP2 Subunit, PLoS ONE, Public Library of Science, 2012, 7, e42870
Berthoud, T. K.; Hamill, M.; Lillie, P. J.; Hwenda, L.; Collins, K. A.; Ewer, K. J.; Milicic, A.; Poyntz, H. C.; Lambe, T. & Fletcher, H. A.
Potent CD8+ T-cell immunogenicity in humans of a novel heterosubtypic influenza A vaccine, MVA- NP+ M1, Clinical infectious diseases, Oxford University Press, 2011, 52, 1-7
Bathurst, I. C.
Protein expression in yeast as an approach to production of recombinant malaria antigens, The American journal of tropical medicine and hygiene, ASTMH, 1994, 50, 20-26
Breunig, K. D.
Multicopy plasmids containing the gene for the transcriptional activator LAC9 are not tolerated by K. lactis cells, Current genetics, Springer, 1989, 15, 143-148
de Silva; Chandimal, U.; Tanaka, H.; Nakamura, S.; Goto, N. & Yasunaga, T. A comprehensive analysis of reassortment in influenza A virus, Biology open, The Company of Biologists Ltd, 2012, 1, 385-390
Eterradossi, N.; Toquin, D.; Abbassi, H.; Rivallan, G.; Cotte, J. & Guittet, M. Passive Protection of Specific Pathogen Free Chicks Against Infectious Bursal Disease by In-Ovo Injection of Semi-Purified Egg-Yolk Antiviral Immunoglobulins, Zoonoses and Public Health, Wiley Online Library, 1997, 44, 371-383
Gellissen, G. & Hollenberg, C. P.
Application of yeasts in gene expression studies: a comparison of Saccharomyces cerevisiae, Hansenula polymorpha and Kluyveromyces lactis-a review, Gene, Elsevier, 1997, 190, 87-97
Gödecke, A.; Zachariae, W.; Arvanitidis, A. & Breunig, K. D.
Coregulation of the Kluyveromyces lactis lactose permease and β-galactoidase genes is achieved by interaction of multiple LAC9 binding sites in a 2.6 kbp divergnent promoter, Nucleic acids research, Oxford University Press, 1991, 19, 5351-5358
Granzow, H.; Birghan, C.; Mettenleiter, T. C.; Beyer, J.; Köllner, B. & Mundt, E. A second form of infectious bursal disease virus-associated tubule contains VP4., Journal of virology, Am Soc Microbiol, 1997, 71, 8879-8885
Kasanga, C. J.; Yamaguchi, T.; Wambura, P. N.; Maeda-Machang'u, A. D.; Ohya, K. & Fukushi, H.
Molecular characterization of infectious bursal disease virus (IBDV): diversity of very virulent IBDV in Tanzania, Archives of virology, Springer, 2007, 152, 783-790
Kirchenbaum, G. A. & Ross, T. M.
Eliciting broadly protective antibody responses against influenza, Current opinion in immunology, Elsevier, 2014, 28, 71-76
Kirunda, H.; Erima, B.; Tumushabe, A.; Kiconco, J.; Tugume, T.; Mulei, S.; Mimbe, D.; Mworozi, E.; Bwogi, J. & Luswa, L.
Prevalence of influenza A viruses in livestock and free-living waterfowl in Uganda, BMC veterinary research, BioMed Central, 2014, 10, 50
Krammer, F. & Palese, P.
Influenza virus hemagglutinin stalk-based antibodies and vaccines, Current opinion in virology, Elsevier, 2013, 3, 521-530
Krijger, J.-J.; Baumann, J.; Wagner, M.; Schulze, K.; Reinsch, C.; Klose, T.; Onuma, O. F.; Simon, C.; Behrens, S.-E. & Breunig, K. D.
A novel, lactase-based selection and strain improvement strategy for recombinant protein expression in Kluyveromyces lactis, Microbial Cell Factories, 2012, 11, 112
Kumar, K.; Singh, K. C. P. & Prasad, C. B.
Immune responses to intermediate strain IBD vaccine at different levels of maternal antibody in broiler chickens, Tropical animal health and production, Springer, 2000, 32, 357-360
Negash, T.; Gelaye, E.; Petersen, H.; Grummer, B. & Rautenschlein, S.
Molecular evidence of very virulent infectious bursal disease viruses in chickens in Ethiopia, Avian diseases, BioOne, 2012, 56, 605-610
Rautenschlein, S.; Kraemer, C. H.; Vanmarcke, J. & Montiel, E.
Protective efficacy of intermediate and intermediate plus infectious bursal disease virus (IBDV) vaccines against very virulent IBDV in commercial broilers, Avian diseases, BioOne, 2005, 49, 231-237
Remington's Practice of Pharmacy, 13. Ausgabe und J. of. Pharmaceutical Science & Technology, Vol. 52, Nr. 5, Sept-Okt., S. 238-311
Ridpath, J. F.
Emerging pestiviruses infecting domestic and wildlife hosts, Animal Health Research Reviews, Cambridge University Press, 2015, 16, 55-59
RKI, Influenza (Teil 2): Erkrankungen durch zoonotische Influenzaviren, 2016 Schrauwen, E. J. A. & Fouchier, R. A. M.
Host adaptation and transmission of influenza A viruses in mammals, Emerging microbes & infections, Nature Publishing Group, 2014, 3, e9
Short, K. R.; Richard, M.; Verhagen, J. H.; van Riel, D.; Schrauwen, E. J. A.; van den Brand, J. M. A.; Mänz, B.; Bodewes, R. & Herfst, S.
One health, multiple challenges: The inter-species transmission of influenza A virus, One health, Elsevier, 2015, 1, 1-13
Steel, J.; Lowen, A. C.; Wang, T. T.; Yondola, M.; Gao, Q.; Haye, K.; García-Sastre, A. & Palese, P.
Influenza virus vaccine based on the conserved hemagglutinin stalk domain, MBio, Am Soc Microbiol, 2010, 1, e00018-10
WHO, Influenza (Seasonal), 2016
WHO, Biologicals, Influenza, 2017

## Patentansprüche

1. *Kluyveromyces lactis (K. lactis)* Stamm zur gezielten Klonierung Fremdantigen- und/oder Fremdprotein codierender Nukleinsäuren in das Hefegenom des *K. lactis* Stammes, **gekennzeichnet dadurch, dass** der *K*. *lactis* Stamm zusätzlich zum *KlLAC4-*Locus am *KlURA3-20-Locus* (*KLLA0E22771g*) und/oder am *KlMETS-1-Locus (KLLA0B03938g)* integrierte Expressionskassetten für Fremdantigene aufweist, wobei die Expressionskassetten den *K. lactis LAC4-12* Promotor (P*LAC4-12*) bzw. Varianten dieses Promotors, einschließlich der intergenischen Region zwischen *LAC12* und *LAC4,* die Antigen- oder Fremdprotein-codierende Region und den *AgTEF1* Terminator enthalten, und wobei die Varianten *K. lactis LAC4-12* Promotor (*PLAC4-12*) ausgewählt sind aus
• einem *LAC4-12* Promotor mit veränderte r Promotorstruktur, die unter nicht-induzierten Bedingungen keine oder eine nur geringe Fremdproteinexpression zulässt, **dadurch gekennzeichnet, dass** die *basal control region* (BCR) des Promotors *PLAC4-12* zwischen -1065 und -1540 (LR2 Deletion; P*LAC4-12-LR2'*(SEQ ID Nr.:2)) deletiert ist; und
• einem *LAC4-12* Promotor mit veränderte Promotorstruktur, die eine Modulation der Fremdproteinexpression ermöglicht, **dadurch gekennzeichnet dass** die Anzahl der Bindungsstellen für den Aktivator KIGal4 des Promotors ("*upstream activating sequences"* 1, 2 und 4, 5) variiert und entweder 1, 2, 3 oder 4 KIGal4 Bindungsstellen vorhanden sind.

2. *K. lactis* Stamm nach Anspruch 1, **dadurch gekennzeichnet, dass** am *KILAC4-Locus* oder am *KlURA3-20-* oder am *KlMET5-1-* Locus der resultierenden *K. lactis* Stämme mehrere Kopien einer Fremdantigen-codierenden Nukleinsäure über Tandem- oder Mehrfach- Expressionskassetten inseriert sind; oder
dass am *KILAC4-Locus* und/oder am *KIURA3-20-* und/oder am *KIMET5-1-* Locus eine oder mehrere Kopien verschiedener Fremdantigen-codierender Nukleinsäuren über Einfach-, Tandem- oder Mehrfach- Expressionskassetten inseriert sind.

3. *K. lactis* Stamm nach einem der Ansprüche 1 oder 2, **dadurch gekennzeichnet, dass** an den *KILAC4* und *KIURA3-20* loci des *K. lactis* Stammes die codierenden Gene der Fremdantigene Influenza A/Puerto Rico/8/1934(H1N1) HA und Influenza A/Puerto Rico/8/1934(H1N1) M1 inseriert sind und exprimiert werden.

4. *K. lactis* Stamm nach einem der vorherigen Ansprüche, **dadurch gekennzeichnet, dass** der *K*. *lactis* Stamm zusätzlich zum genomischen *KIGAL4* Gen noch eine zweite ektopische Kopie des *KIGAL4* Gens enthält.

5. *K. lactis* Stamm nach einem der vorherigen Ansprüche, **dadurch gekennzeichnet, dass** die Genfunktion der Allele *Kllac4, Klura3-20* und *Klmet5-1* wiederhergestellt ist und der *K. lactis* Stamm prototroph ist.

6. *K. lactis* Stamm nach einem der vorherigen Ansprüche, **dadurch gekennzeichnet, dass** an den Loci *KILAC4, KIURA3-20* und *KIMet5-1* des *K*. *lactis* Stammes die Gene der Fremdantigene BVDV E2 ectodomain (Typ 1, CP7), BVDV E2 ectodomain (Typ 2, New York 93) und BVDV Npro-NS3 (Typ1, CP7) inseriert sind.

7. *K. lactis* Stamm, ausgewählt aus den Stämmen:
| | |
|---|---|
| VAK1283 | DSM 32697; |
| VAK1395 | DSM 32706 und |
| VAK1400 | DSM 32698 |

8. Pharmazeutische Zusammensetzung, enthaltend einen *K. lactis* Stamm nach einem der Ansprüche 1 bis 7.

9. *K. lactis* Stamm nach einem der Ansprüche 1 bis 7 zur Verwendung in der Vakzinierung.

10. *K. lactis* Stamm zur Verwendung nach Anspruch 9 **dadurch gekennzeichnet, dass** die Vakzinierung eine protektive Vakzinierung ist.

11. *K. lactis* Stamm zur Verwendung nach einem der Ansprüche 9 oder 10, umfassend die Verabreichung eines *K*. *lactis* Stammes nach einem der Ansprüche 1 bis 7 an ein Subjekt in einer Menge, die ausreichend ist, um in dem Subjekt eine protektive Immunantwort gegen ein oder mehrere Fremdantigene auszulösen.

12. *K. lactis* Stamm zur Verwendung nach einem der Ansprüche 9 bis 11, **dadurch gekennzeichnet, dass** der *K*. *lactis* Stamm subkutan, intramuskulär oder oral/mukosal verabreicht wird.

13. *K. lactis* Stamm zur Verwendung nach einem der Ansprüche 9 bis 11, **dadurch gekennzeichnet, dass** der *K*. *lactis* Stamm in einer einmaligen Anwendung/Immunisierung ("*one shot*") oder in einer zweifachen Anwendung/Immunisierung (*"prime-boost*") eine protektive Immunantwort gegen ein Pathogen auslöst.

14. *K. lactis* Stamm zur Verwendung nach nach einem der Ansprüche 9 bis 11, **dadurch gekennzeichnet, dass** der *K*. *lactis* Stamm in einer einmaligen Anwendung/Immunisierung ("*one shot")* oder in einer zweifachen Anwendung/Immunisierung ("*prime-boost*") eine gegen verschiedene Varianten eines Pathogens kreuzprotektive Immunantwort auslöst.

15. *K. lactis* Stamm nach einem der Ansprüche 9 bis 11, **dadurch gekennzeichnet, dass** der *K. lactis* Stamm in einer einmaligen Anwendung/Immunisierung ("*one shot*") oder in einer zweifachen Anwendung/Immunisierung ("*prime-boost*") eine protektive Immunantwort gegen verschiedene Pathogene auslöst.

## Claims

1. *Kluyveromyces lactis* (*K. lactis*) strain for targeted cloning of foreign antigen-encoding and/or foreign protein-encoding nucleic acids into the yeast genome of the *K. lactis* strain, **characterized in that** the *K. lactis* strain has integrated expression cassettes for foreign antigens at the *KlURA3-20* locus *(KLLA0E22771g)* and/or at the *KlMET5-1* locus (*KLLA0B03938g*) in addition to the *K1LAC4* locus, wherein the expression cassettes contain the *K. lactis LAC4-12* promoter (P*LAC4-12*) or variants of said promoter, including the intergenic region between *LAC12* and *LAC4,* the antigen-encoding or foreign protein-encoding region and the *AgTEF1* terminator, and wherein the *K. lactis LAC4-12* promoter (*PLAC4-12*) variants are selected from
• a *LAC4-12* promoter having a modified promoter structure that allows only slight foreign protein expression or none under noninduced conditions, **characterized in that** the basal control region (BCR) of the promoter P*LAC4-12* between -1065 and -1540 (LR2 deletion; P*LAC4-12-LR2*' (SEQ ID No.: 2)) is deleted;
and
• a *LAC4-12* promoter having a modified promoter structure that allows modulation of foreign protein expression, **characterized in that** the number of binding sites for the activator KlGal4 of the promoter ("upstream activating sequences" 1, 2 and 4, 5) varies and 1, 2, 3 or 4 KlGal4-binding sites are present.

2. *K. lactis* strain according to Claim 1, **characterized in that** multiple copies of a foreign antigen-encoding nucleic acid are inserted via tandem expression cassettes or multi-expression cassettes at the *K1LAC4* locus or at the *KlURA3-20* locus or at the *KlMET5-1* locus of the resultant *K. lactis* strains; or
**in that** one or more copies of different foreign antigen-encoding nucleic acids are inserted via single expression cassettes, tandem expression cassettes or multi-expression cassettes at the *K1LAC4* locus and/or at the *KlURA3-20* locus and/or at the *KlMET5-*1 locus.

3. *K. lactis* strain according to either of Claims 1 and 2, **characterized in that** the encoding genes of the foreign antigens influenza A/Puerto Rico/8/1934(H1N1) HA and influenza A/Puerto Rico/8/1934(H1N1) M1 are inserted at the *K1LAC4* and *KlURA3-20* loci of the *K. lactis* strain and are expressed.

4. *K. lactis* strain according to any of the preceding claims, **characterized in that** the *K. lactis* strain contains, in addition to the genomic *KlGAL4* gene, additionally a second ectopic copy of the *KlGAL4* gene.

5. *K. lactis* strain according to any of the preceding claims, **characterized in that** the gene function of the alleles *Kllac4, Klura3-20* and *Klmet5-1* is restored and the *K. lactis* strain is prototrophic.

6. *K. lactis* strain according to any of the preceding claims, **characterized in that** the genes of the foreign antigens BVDV E2 ectodomain (type 1, CP7), BVDV E2 ectodomain (type 2, New York 93) and BVDV Npro-NS3 (type 1, CP7) are inserted at the loci *K1LAC4, K1URA3-20* and *KlMet5-1* of the *K. lactis* strain.

7. *K. lactis* strain selected from the strains:
| | |
|---|---|
| VAK1283 | DSM 32697; |
| VAK1395 | DSM 32706 and |
| VAK1400 | DSM 32698 |

8. Pharmaceutical composition containing a *K. lactis* strain according to any of Claims 1 to 7.

9. *K. lactis* strain according to any of Claims 1 to 7 for use in vaccination.

10. *K. lactis* strain for use according to Claim 9, **characterized in that** the vaccination is a protective vaccination.

11. *K. lactis* strain for use according to either of Claims 9 and 10, comprising administering a *K. lactis* strain according to any of Claims 1 to 7 to a subject in an amount sufficient for triggering a protective immune response against one or more foreign antigens in the subject.

12. *K. lactis* strain for use according to any of Claims 9 to 11, **characterized in that** the *K. lactis* strain is administered subcutaneously, intramuscularly or orally/mucosally.

13. *K. lactis* strain for use according to any of Claims 9 to 11, **characterized in that** the *K. lactis* strain triggers a protective immune response against a pathogen in a single use/immunization ("one shot") or in a double use/immunization ("prime-boost").

14. *K. lactis* strain for use according to any of Claims 9 to 11, **characterized in that** the *K. lactis* strain triggers a cross-protective immune response against different variants of a pathogen in a single use/immunization ("one shot") or in a double use/immunization ("prime-boost").

15. *K. lactis* strain according to any of Claims 9 to 11, **characterized in that** the *K. lactis* strain triggers a protective immune response against different pathogens in a single use/immunization ("one shot") or in a double use/immunization ("prime-boost").

## Revendications

1. Souche de *Kluyveromyces lactis* (*K. lactis*) pour le clonage ciblé d'acides nucléiques codant pour des antigènes étrangers et/ou des protéines étrangères dans le génome de levure de la souche de *K. lactis,* **caractérisée en ce que** la souche de *K*. *lactis* comprend des cassettes d'expression pour antigènes étrangers intégrés, en plus du locus *KILAC4,* sur le locus *KIURA3-20 (KLLA0E22771g)* et/ou sur le locus *KIMET5-1* (*KLLA0B03938g*)*,* les cassettes d'expression contenant le promoteur *LAC4-12* de *K*. *lactis* (P*LAC4-12*) ou des variants de ce promoteur, y compris de la région intergénique entre *LAC12* et *LAC4,* la région codant pour des antigènes ou des protéines étrangères et le terminateur *AgTEF1*, les variants du promoteur *LAC4-12* de *K. lactis* (P*LAC4-*12) étant choisis parmi
• un promoteur *LAC4-12* ayant une structure promotrice modifiée, qui dans des conditions non induites n'autorise aucune expression de protéines étrangères ou une expression seulement faible des protéines étrangères, **caractérisée en ce que** la *basal control region* (BCR) du promoteur P*LAC4-12* est délétée entre - 1065 et -1540 (délétion LR2 ; P*LAC4-12-LR2*' (SEQ ID NO: 2)) ; et
• un promoteur *LAC4-12* ayant une structure promotrice modifiée, qui autorise une modulation de l'expression des protéines étrangères, **caractérisée en ce que** le nombre des sites de liaison pour l'activateur KIGal4 du promoteur (« *upstream activating séquences* » 1, 2 et 4, 5) varie, et 1, 2, 3 ou 4 sites de liaison KIGal4 étant présents.

2. Souche de *K. lactis* selon la revendication 1, **caractérisée en ce que** plusieurs copies d'un acide nucléique codant pour des antigènes étrangers sont insérées par l'intermédiaire de cassettes d'expression en tandem ou multiples sur le locus *KILAC4* ou sur le locus *KIURA3-20* ou *KIMET5-1* des souches de *K. lactis* résultantes ; ou
**en ce qu'**une ou plusieurs copies de différents acides nucléiques codant pour des antigènes étrangers sont insérées par l'intermédiaire de cassettes d'expression simples, en tandem ou multiples, sur le locus *KILAC4* et/ou sur le locus *KIURA3-20* et/ou *KIMET5-1.*

3. Souche de *K. lactis* selon l'une des revendications 1 ou 2, **caractérisée en ce que** les gènes codant des antigènes étrangers Influenza A/Puerto Rico/8/1934(H1N1) HA et Influenza A/Puerto Rico/8/1934(H1N1) M1 sont insérés et exprimés dans les loci *KILAC4* et *KIURA3-20* de la souche de *K. lactis.*

4. Souche de *K. lactis* selon l'une des revendications précédentes, **caractérisée en ce que** la souche de *K. lactis* contient encore, en plus du gène *KIGAL4* génomique, une deuxième copie ectopique du gène *KIGAL4.*

5. Souche de *K. lactis* selon l'une des revendications précédentes, **caractérisée en ce que** la fonction génique des allèles *KIlac4, KIura3-20* et *KImet5-1* est rétablie, et la souche de *K. lactis* est prototrophe.

6. Souche de *K. lactis* selon l'une des revendications précédentes, **caractérisée en ce que** les gènes des anticorps étrangers ectodomaine E2 de BVDV (Type 1, CP7), ectodomaine E2 de BVDV (Type 2, New York 93) et Npro-NS3 de BVDV (Type 1, CP7), sont insérés sur les loci *KILAC4, KIURA3-20* et *KIMet5-1* de la souche de *K. lactis.*

7. Souche de *K. lactis* choisie parmi les souches :
| | |
|---|---|
| VAK1283 | DSM 32697 ; |
| VAK1395 | DSM 32706 et |
| VAK1400 | DSM 32698 |

8. Composition pharmaceutique contenant une souche de *K*. *lactis* selon l'une des revendications 1 à 7.

9. Souche de *K. lactis* selon l'une des revendications 1 à 7 pour une utilisation en vaccination.

10. Souche de *K. lactis* pour une utilisation selon la revendication 9, **caractérisée en ce que** la vaccination est une vaccination protective.

11. Souche de *K. lactis* pour une utilisation selon l'une des revendications 9 ou 10, comprenant l'administration d'une souche de *K. lactis* selon l'une des revendications 1 à 7 à un sujet en une quantité qui est suffisante pour déclencher chez le sujet une réponse immunitaire protective contre un ou plusieurs antigènes étrangers.

12. Souche de *K. lactis* pour une utilisation selon l'une des revendications 9 à 11, **caractérisée en ce que** la souche de *K*. *lactis* est administrée par voie sous-cutanée, intramusculaire ou orale/muqueuse.

13. Souche de *K. lactis* pour une utilisation selon l'une des revendications 9 à 11, **caractérisée en ce que** la souche de *K*. *lactis* déclenche une réponse immunitaire protective contre un pathogène lors d'une utilisation/immunisation unique (« *one shot* ») ou d'une utilisation/immunisation en deux fois (« *prime-boost* »)*.*

14. Souche de *K. lactis* pour une utilisation selon l'une des revendications 9 à 11, **caractérisée en ce que** la souche de *K*. *lactis* déclenche une réponse immunitaire à protection croisée contre différents variants d'un pathogène lors d'une utilisation/immunisation unique (« *one shot* ») ou d'une utilisation/immunisation en deux fois (« *prime-boost* »)*.*

15. Souche de *K. lactis* selon l'une des revendications 9 à 11, **caractérisée en ce que** la Souche de K. lactis déclenche une réponse immunitaire protective contre différents pathogènes lors d'une utilisation/immunisation unique (« *one shot* ») ou d'une utilisation/immunisation en deux fois (« *prime-boost* »)*.*
